# EUROPEAN PATENT SPECIFICATION

(11) **EP 0 961 775 B1**
(45) Date of publication and mention of the grant of the patent: **14.07.2004**
(21) Application number: 97911684.5
(22) Date of filing: 15.10.1997
(51) Int. Cl.: C07H 19/16, C07H 19/04, C07H 19/24, A61K 31/70

(54) **PURINE L-NUCLEOSIDES, ANALOGS AND USES THEREOF**
PURIN-L-NUKLEOSIDE, DEREN ANALOGA UND VERWENDUNGEN
ANALOGUES DE NUCLEOSIDES L PURINE ET LEURS UTILISATIONS

(30) Priority: 16.10.1996 US 28586 P; 23.04.1997 US 43974 P; 12.08.1997 US 55487 P
(43) Date of publication of application: 08.12.1999
(60) Divisional application: 00118428.2
(73) Proprietor: ICN Pharmaceuticals, Inc., Costa Mesa, CA 92626 (US)
(72) Inventor: WANG, Guangyi, Irvine, CA 92715 (US); TAM, Robert, Irvine, CA 92606 (US); AVERTT, Deveron, Irvine, CA 92612 (US)
(74) Representative: Viering, Jentschura & Partner
(86) International application number: PCT/US1997/018387
(87) International publication number: WO 1998/016184

(56) References cited:
- EP-A- 0 285 884
- EP-A- 0 343 945
- WO-A-89/05649
- WO-A-98/05817
- US-A- 5 248 776
- US-A- 5 473 063
- US-A- 5 559 101
- US-A- 5 561 120
- US-A- 5 565 438
- US-A- 5 567 688
- US-A- 5 567 689
- US-A- 5 587 362
- US-A- 5 599 796
- US-A- 5 627 160
- US-A- 5 631 239
- US-A- 5 672 594
- HOLY A ET AL: "OLIGONUCLEOTIDIC COMPOUNDS. XXXIV. PREPARATION OF SOME BETA-L-RIBONUCLEOSIDES, THEIR 2'(3'-CYCLIC PHOSPHATES" COLLECTION OF CZECHOSLOVAK CHEMICAL COMMUNICATIONS,ACADEMIC PRESS, LONDON,GB, vol. 34, 1969, pages 3383-3401, XP001007202 ISSN: 0010-0765
- ANTIMICROBIAL AGENTS AND CHEMOTHERAPY, April 1994, Vol. 38, No. 4, VAN DRAANEN et al., "Influence of Stereochemistry on Antiviral Activities and Resistance Profiles of Dideoxycytidine Nucleosides", pages 868-871, XP002947715

## Description

### FIELD OF THE INVENTION

The present invention relates to the field of L-nucleosides.

### BACKGROUND OF THE INVENTION

The last few decades have seen significant efforts expended in exploring possible uses of D-nucleoside analogs as antiviral agents. Some of this work has borne fruit, and a number of nucleoside analogs are currently being marketed as antiviral drugs, including the HIV reverse transcriptase inhibitors (AZT, ddI, ddC, d4T, and 3TC).

A variety of purine D-nucleoside analogs have also been explored in search of immuno-modulators. Guanosine analogs having substituents at the 7- and/or 8-positions, for example, have been shown to stimulate the immune system (for a review, see: Weigle, W.O. CRC *Crit. Rev. Immunol.* **1987,** 7, 285; Lin et al. *J. Med. Chem.* **1985,** 28, 1194-1198; Reitz, et al. *J. Med. Chem.* **1994,** 37, 3561-3578, Michael et al. *J. Med. Chem.* **1993,** 36, 3431-3436). Certain 3-β-D-ribofuranosylthiazolo[4,5-d]pyrimidines have also demonstrated significant immunoactivity, including murine spleen cell proliferation and *in vivo* activity against Semliki Forest virus (Nagahara, et al. *J. Med. Chem.* **1990,** 33, 407-415; Robins et al. U.S. Patent 5,041,426). In other research, 7-Deazaguanosine and analogs have been shown to exhibit antiviral activity in mice against a variety of RNA viruses, even though the compound lacks antiviral properties in cell culture. 3-Deazaguanine nucleosides and nucleotides have also demonstrated significant broad spectrum antiviral activity against certain DNA and RNA viruses (Revankar et al. *J. Med. Chem.* **1984,** 27, 1389-1396). Certain 7- and 9-deazaguanine C-nucleosides exhibit the ability to protect mice against a lethal challenge of Semliki Forest virus (Girgis et al. *J. Med. Chem.* **1990,** 33, 2750-2755). Certain 6-sulfenamide and 6-sulfmamide purine nucleosides have demonstrated significant antitumor activity (Robins et al. U.S. Patent 4,328,336). Certain pyrimido[5,4-D]pyrimidine nucleosides were effective in treatment against L1210 in BDF1 mice (Robins et al. U.S. Patent 5,041,542), and there, the antiviral and antitumor activities of the above mentioned nucleosides were suggested to be the results of the their role as immunomodulators (Bonnet et al. *J med. Chem*. **1993**, *36*, 635-653).

One possible target of immunomodulation involves stimulation or suppression of Th1 and Th2 lymphokines. Type I (Th1) cells produce interleukin 2 (IL-2), tumor necrosis factor (TNFα) and interferon gamma (IFNγ) and they are responsible primarily for cell-mediated immunity such as delayed type hypersensitivity and antiviral immunity. Type 2 (Th2) cells produce interleukins, IL4, IL-5, IL-6, IL-9, IL-10 and IL-13 and are primarily involved in assisting humoral immune responses such as those seen in response to allergens, e.g. IgE and IgG4 antibody isotype switching (Mosmann, 1989, *Annu Rev Immunol,* 7:145-173). D-guanosine analogs have been shown to elicit various effects on lymphokines IL-1, IL-6, IFNα and TNFα (indirectly) *in vitro* (Goodman, 1988, *Int J Immunopharmacol,* **10**, 579-88) and *in vivo* (Smee et al., 1991, *Antiviral Res* 15: *229*). However, the ability of the D-guanosine analogs such as 7- thio-8-oxoguanosine to modulate Type I or Type 2 cytokines directly in T cells was ineffective or had not been described.

Thus, there remains a need for novel L-nucleoside analogs, including novel purine L-nucleoside analogs. There is a particular need for novel purine L-nucleosides which have immunomodulatory activity, and especially for novel purine L-nucleosides which modulate Th1 and Th2 activity.

US Patent No. 5,559,101 discloses α and β L-Ribofuranosylnucleosides, processes for their preparation, pharmaceutical compositions containing them and methods for using these compounds as anticancer, antiviral, antiparasitic, antifungal, antibacterial, antimicrobial agents and antitumor agents to treat various diseases in mammals.

### BRIEF DESCRIPTION OF THE INVENTION

The present invention is directed to novel purine L-nucleoside compounds, their therapeutic uses and synthesis.

In one aspect of the invention, there are provided purine L-nucleoside analogs of general Formulas I-A, I-B, I-C, I-D, and I-E as described in more detail on pages 7-10. All of these L-nucleoside analogs can be summarized under general formula I which, however, further includes L-nucleoside analogs that do not fall under the scope of the present invention. 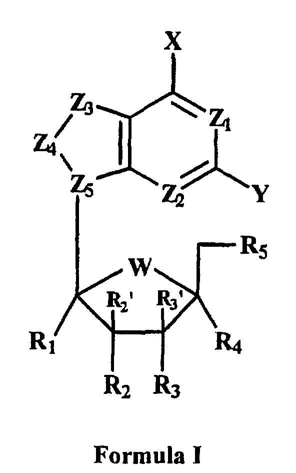 wherein R₁, R₂, R₃, R₄, R₅, R₂' and R₃' are independently selected from the group consisting of H, OH, NH₂, F, Cl, Br, I, N₃, -CN, -OR', -NR'₂, -SR', -NHNH₂, -NHOH, CHO, COOR', CONR'₂, alkyl, alkenyl, alkylnyl, aryl, aralkyl, substituted alkyl, substituted alkenyl, substituted alkynyl, substituted aryl, substituted aralkyl, where the substituent is selected from F, Cl, Br, I, N₃, -CN, -OR", NO₂, -NR''₂, SR", -NHNH₂, -NHOH, COOR", CONR''₂ and where R' and R" are H, alkyl, alkenyl, alkynyl, aryl, aralkyl;
W = O, S, CH₂, Se;
Z₁, Z₂ are independently selected from N, C, CH;
Z₃, Z₄, Z₅ are independently selected from the group consisting of -CR-, -NR-, -O-, -S-, -Se-, -C=O, -C=S, -S=O, -CR=CR-, -CR=N-, -N=N-, where R is selected from the group consisting of H, F, Cl, Br, I, N₃, -CN, -OR', -NR'₂, -SR', -NHNH₂, -NHOH, -NO₂, CHO, COOR', CONH₂, -C(O)-NH₂, -C(S)-NH₂, -C(NH)-NH₂, -C(NOH)-NH₂, =O, =NH, =NOH, =NR, alkyl, alkenyl, alkylnyl, aryl, aralkyl, substituted alkyl, substituted alkenyl, substituted alkynyl, substituted aryl, substituted aralkyl, where the substituent is selected from H, -OH, NH₂, F, Cl, Br, I, N₃ -CN, -COOR", -CONR"₂, -OR", -NR"_{2'} -SR", -NHNH₂, -NHOH, -NO₂, and R', R" are H, alkyl, alkenyl, alkynyl, aryl, aralkyl, acetyl, acyl, sulfonyl;
The Chemical bond between Z₃ and Z₄ or Z₄ and Z₅ is selected from C-C, C=C, C-N, C=N, N-N, N=N, C-S, N-S;
X and Y are independently selected from the group consisting of H, OH, NH₂, F, Cl, Br, I, N₃, -S-NH₂, -S(O)-NH₂, -S(O₂)-NH₂, -CN, -COOR', -CONR'₂, -OR', -NR'₂, -SR', -NHNH₂, -NHOH, alkyl, alkenyl, alkylnyl, aryl, aralkyl, substituted alkyl, substituted alkenyl, substituted alkynyl, substituted aryl, substituted aralkyl, where the substituent is selected from F, Cl, Br, I, N₃, -CN, -OR", NO₂, -NR''₂, SR'', -NHNH₂, -NHOH, and R', R'' are H, alkyl, alkenyl, alkynyl, aryl, aralkyl.

In another aspect of the invention, a pharmaceutical composition comprises a therapeutically effective amount of a compound of Formulas I-A, I-B, I-C, I-D, and I-E as described in more detail on pages 7-10, or a pharmaceutically acceptable ester or salt thereof admixed with at least one pharmaceutically acceptabte carrier.

In yet another aspect of the invention, a compound according to Formulas I-A, I-B, I-C, I-D, and I-E as described in more detail on pages 7-10 is used in the treatment of any condition which responds positively to administration of the compound, and according to any formulation and protocol which achieves the positive response. Among other things it is contemplated that compounds of Formulas I-A, I-B, I-C, I-D, and I-E as described in more detail on pages 7-10 may be used to treat an infection, an infestation, a cancer, tumor or other neoplasm, or an autoimmune disease.

### BRIEF DESCRIPTION OF THE FIGURES

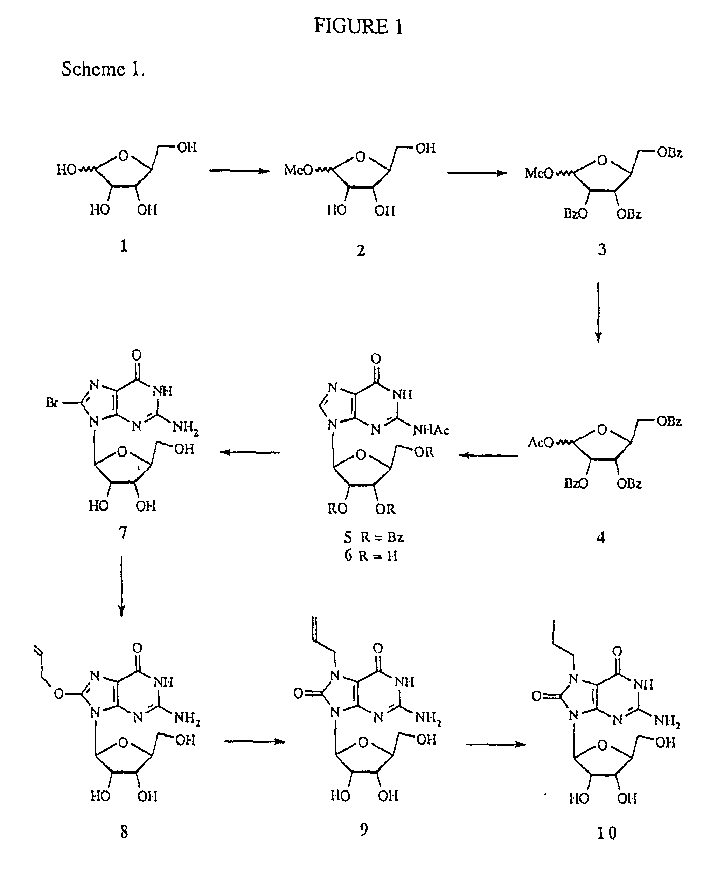

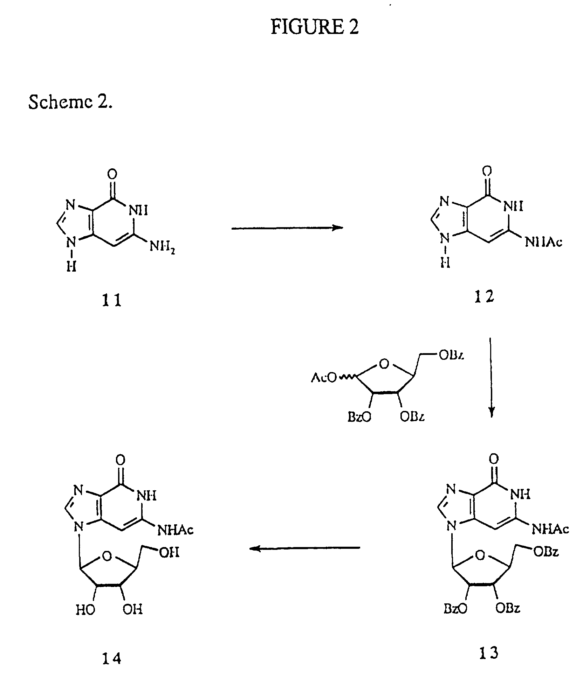

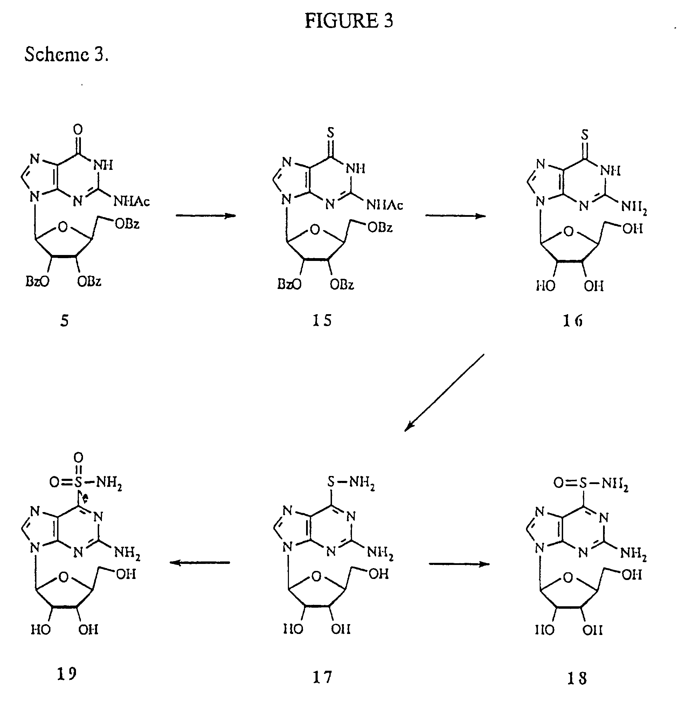

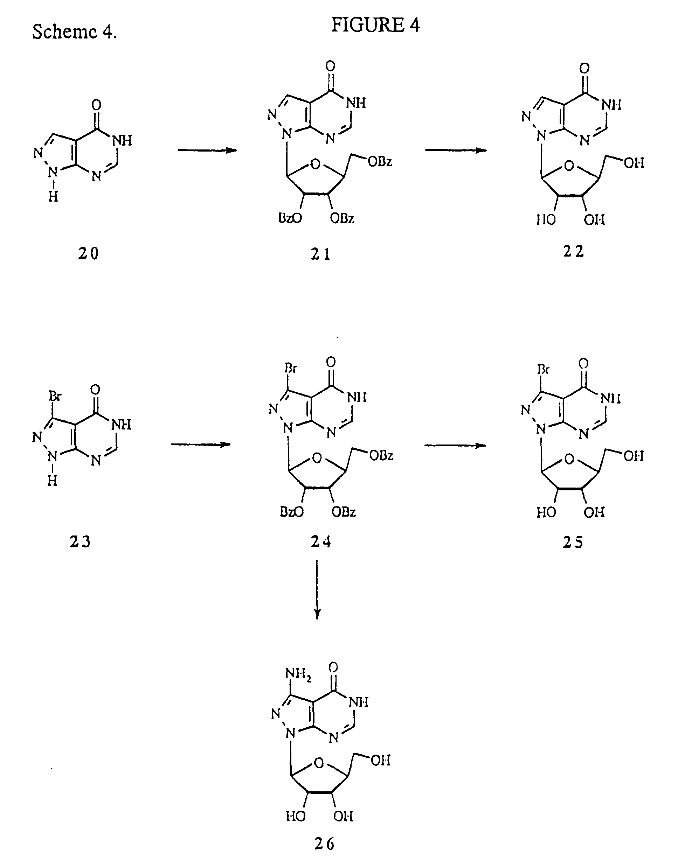

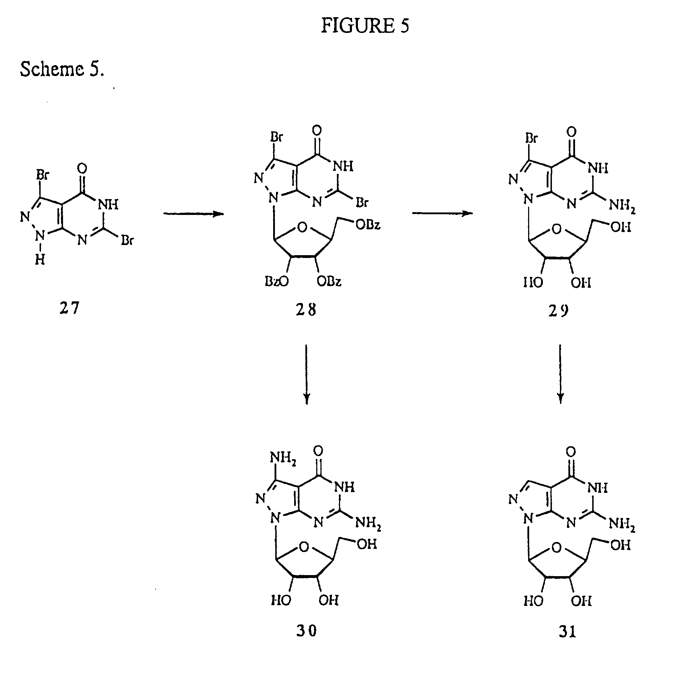

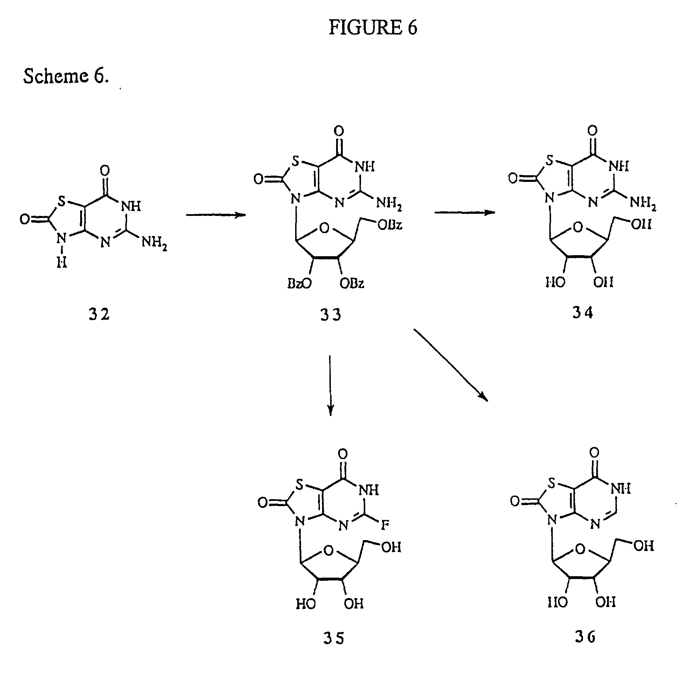

Figures 1-6 (Schemes 1-6) depict synthetic chemical steps which may be used to synthesize the compounds according to the present invention. Schemes pertaining to the synthesis of a particular composition are referenced in the examples set forth herein.
Figure 7 is a graphical depiction of exemplary L-guanosine analogs on Th1 and Th2.

### DETAILED DESCRIPTION

Where the following terms are used in this specification, they are used as defined below.

The term "nucleoside" refers to a compound composed of any pentose or modified pentose moiety attached to a specific position of a heterocycle or to the natural position of a purine (9-position) or pyrimidine (1-position) or to the equivalent position in an analog.

The term "nucleotide" refers to a phosphate ester substituted on the 5'-position of a nucleoside.

The term "heterocycle" refers to a monovalent saturated or unsaturated carbocyclic radical having at least one hetero atom, such as N, O or S, within the ring each available position of which can be optionally substituted, independently, with, e.g., hydroxy, oxo, amino, imino, lower alkyl, bromo, chloro and/or cyano. Included within this class of substituents are purines, pyrimidines.

The term "purine" refers to nitrogenous bicyclic heterocycles.

The term "pyrimidine" refers to nitrogenous monocyclic heterocycles.

The term "D-nucleosides" that is used in the present invention describes to the nucleoside compounds that have a D-ribose sugar moiety (e.g., Adenosine).

The term "L-nucleosides" that is used in the present invention describes to the nucleoside compounds that have an L-ribose sugar moiety.

The term "L-configuration" is used throughout the present invention to describe the chemical configuration of the ribofuranosyl moiety of the compounds that is linked to the nucleobases. The L-configuration of the sugar moiety of compounds of the present invention contrasts with the D-configuration of ribose sugar moieties of the naturally occurring nucleosides such as cytidine, adenosine, thymidine, guanosine and uridine.

The term "C-nucleosides" is used throughout the specification to describe the linkage type that formed between the ribose sugar moiety and the heterocyclic base. In C-nucleosides, the linkage originates from the C-1 position of the ribose sugar moiety and joins the carbon of the heterocyclic base. The linkage that forms in C-nucleosides are carbon to carbon type.

The term "N-nucleosides" is used throughout the specification to describe the linkage type that formed between the ribose sugar moiety and the heterocyclic base. In N-nucleosides, the linkage originates from the C-1 position of the ribose sugar moiety and joins the nitrogen of the heterocyclic base. The linkage that forms in N-nucleosides are carbon to nitrogen type.

The term "protecting group" refers to a chemical group that is added to, oxygen or nitrogen atom to prevent its further reaction during the course of derivatization of other moieties in the molecule in which the oxygen or nitrogen is located. A wide variety of oxygen and nitrogen protecting groups are known to those skilled in the art of organic synthesis.

The term "lower alkyl" refers to methyl, ethyl, n-propyl, isopropyl, n-butyl, t-butyl, i-butyl or n-hexyl. This term is further exemplified to a cyclic, branched or straight chain from one to six carbon atoms.

The term "aryl" refers to a monovalent unsaturated aromatic carbocyclic radical having a single ring (e.g., phenyl) or two condensed rings (e.g., naphthyl), which can optionally be substituted with hydroxyl, lower alky, chloro, and/or cyano.

The term "heterocycle" refers to a monovalent saturated or unsaturated carbocyclic radical having at least one hetero atom, such as N, O, S, Se or P, within the ring, each available position of which can be optionally substituted or unsubstituted, independently, with e.g., hydroxy, oxo, amino, imino, lower alkyl, bromo, chloro, and/or cyano.

The term "monocyclic" refers to a monovalent saturated carbocyclic radical having at least one hetero atom, such as O, N, S, Se or P, within the ring, each available position of which can be optionally substituted, independently, with a sugar moiety or any other groups like bromo, chloro and/or cyano, so that the monocyclic ring system eventually aromatized [e.g., Thymidine; 1-(2'-deoxy-β-D-erythro-pentofuraosyl)thymine].

The term "immunomodulators" refers to natural or synthetic products capable of modifying the normal or aberrant immune system through stimulation or suppression.

The term "effective amount" refers to the amount of a compound of Formulas I-A, I-B, I-C, I-D, and I-E as described in more detail on pages 7-10 which will restore immune function to normal levels, or increase immune function above normal levels in order to eliminate infection.

The compounds of Formulas I-A, I-B, I-C, I-D, and I-E as described in more detail on pages 7-10 may have multiple asymmetric centers. Accordingly, they may be prepared in either optically active form or as a racemic mixture. The scope of the invention as described and claimed encompasses the individual optical isomers and non-racemic mixtures thereof as well as the racemic forms of the compounds of Formulas I-A, I-B, I-C, I-D, and I-E as described in more detail on pages 7-10.

The term "α" and "β" indicate the specific stereochemical configuration of a substituent at an asymmetric carbon atom in a chemical structure as drawn. The compounds described herein are all in the L-furanosyl configuration.

The term "enantiomers" refers to a pair of stereoisomers that are non-superimposable mirror images of each other. A mixture of a pair of enantiomers, in a 1:1 ratio, is a "racemic" mixture.

The term "isomers" refers to different compounds that have the same formula. "Stereoisomers" are isomers that differ only in the way the atoms are arranged in space.

A "pharmaceutically acceptable salts" may be any salts derived from inorganic and organic acids or bases.

### Compounds

The compounds of the present invention are generally described by Formulas I-A through I-E below.

Formula I-A compounds are 8-substituted α-or β- L-guanosine analogs having the structure: 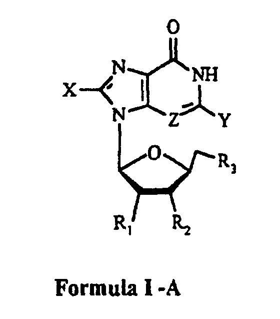 wherein X is selected from R^{x}, F, Cl, Br, I, N₃, -CN, -OR^{x}, -SR^{x}, -NR^{x}₂, -NHNH₂, -NHOH,-CHO, -CONH₂, -COOR^{x}, and -L-A; where R^{x} is selected from alkyl, alkenyl, alkynyl, and aralkyl, acetyl, acyl, sulfonyl; L is a linker and selected from alkyl, alkenyl, alkynyl, and aralkyl; and A is selected from H, -OR', -SR', -NR'₂, -NHNR'₂, -CHO,-COOR', -CONR'₂, where R' is selected from H, Me, Et, allyl, acetyl, -COCF₃;
Y is selected from H, R^{y}, F, Cl, Br, I, N₃, CN, OR^{y}, SR^{y}, NR^{y}₂, where R^{y} is selected from H, alkyl, alkenyl, alkynyl, and aralkyl, acetyl, acyl, sulfonyl;
Z is N or CH; and
R₁, R₂, and R₃ are independently selected from H, -OH, -OAc, -OBz, -OP(O₂)OH.

Formula IB compounds are 7-substituted-8-oxo-α-or β- L-guanosine analogs having structure: 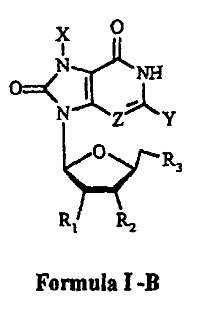 wherein X is selected from H, R^{x}, -NH₂, -CHO, -COOR^{x}, -L-A, where R^{x} is selected from alkyl, alkenyl, alkynyl, and aralkyl, L is a linker and selected from alkyl, alkenyl, alkynyl, and aralkyl; A is selected from H, F, Cl, Br, I, -OR', -SR', -NR'₂, -NHNH₂, -NHOH, N₃, -CHO, -CONH₂, -COOR', -CN, where R' is selected from Me, Et, allyl, acetyl, -COCF₃;
Y is selected from H, R^{y}, F, Cl, Br, I, N₃, -CN, -OR^{y}, -SR^{y}, -NR^{y}, where R^{y} is selected from H, alkyl, alkenyl, alkynyl, and aralkyl, acetyl, acyl, sulfonyl;
Z is N or CH;
R₁, R₂, and R₃ are independently selected from H, -OH, -OAc, -OBz, -OP(O₂)OH, with the proviso that where R₁, R₂, and R₃ are OH, then Z is not N, Y is not NH₂, and X is not propyl.

Formula I-C compounds are 7-deaza-7,8-mono- or disubstituted α-or β- L-guanosine analogs having the structure: 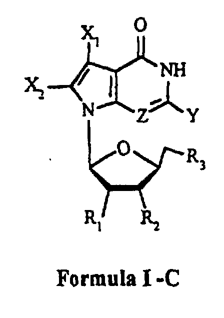 wherein X₁ and X₂ are independently selected from H, R^{x}, F, Cl, Br, I, N₃, -CN, -OR^{x}, -SR^{x}, -NR^{x}₂, -NHNH₂, -NHOH, -CHO, -CONH₂, -COOR^{x}, and -L-A; where R^{x} is selected from alkyl, alkenyl, alkynyl, and aralkyl, acetyl, acyl, sulfonyl; L is a linker and selected from alkyl, alkenyl, alkynyl, and aralkyl; and A is selected from H, -OR',-SR', -NR'₂, -NHNR'₂, -CHO, -COOR', -CONR'₂, where R' is selected from H, Me, Et, allyl, acetyl, -COCF₃;
Y is selected from H, R^{y}, F, Cl, Br, I, N₃, -CN, -OR^{y}, -SR^{y}, -NR^{y}₂, where R^{y} is selected from H, alkyl, alkenyl, alkynyl, and aralkyl, acetyl, acyl, sulfonyl;
Z is N or CH;
R₁, R₂, and R₃ are independently selected from H, -OR, -OAc, -OBz, -OP(O₂)OH.

Formula I-D compounds are 7-deaza-8-aza-7-substituted α-or β-L-guanosine analogs having the structure 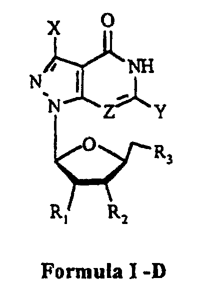 X is selected from H, R^{x}, F, Cl, Br, I, N₃, -CN, -OR^{x}, SR^{x}, -NR^{x}₂, -NHNH₂, -NHOH, -CHO, -CONH₂, -COOR^{x}, and -L-A; where R^{x} is selected from alkyl, alkenyl, alkynyl, and aralkyl, acetyl, acyl, sulfonyl; L is a linker and selected from alkyl, alkenyl, alkynyl, and aralkyl; and A is selected from H, -OR', SR', -NR'₂, -NHNR'₂, -CHO, -COOR',-CONR'₂, where R' is selected from H, Me, Et, allyl, acetyl, -COCF₃;
Y is selected from H, R^{y}, F, Cl, Br, I, N₃, -CN, -OR^{y,} -SR^{y}, -NR^{y}₂, where R^{y} is selected from H, alkyl, alkenyl, alkynyl, and aralkyl, acetyl, acyl, sulfonyl;
Z is N or CH,
R₁, R₂, and R₃ are independently selected from H, -OR, -OAc, -OBz, -OP(O₂)OH.

Formula I-E compounds are thiazolo[4,5-d]pyrimidine α-or β-L-nucleosides having the structure: 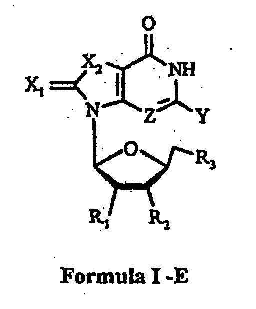 X₁ = O, S, =NH, =NNH₂, =NHOH, =NR^{x} where R^{x} is selected from alkyl alkyenyl, alkynyl, and aralkyl, acyl;
X₂ is S, O, or Se
Y is selected from H, R^{y}, F, Cl, Br, I, N₃, -CN, -OR^{y}, -SR^{y}, -NR^{y}₂, where R^{y} is selected from H, alkyl, alkenyl, alkynyl, and aralkyl, acetyl, acyl, sulfonyl;
Z is N or CH;
R₁, R₂, and R₃ are independently selected from H, -OH, -OAc, -OBz, -OP(O₂)OH, while the proviso that when R₁, R₂, and R₃ are OH then Z is not N, X₁ is not O, X₂ is not S, and Y is not NH₂.

Formula I-F compounds are merely included to illustrate the present invention and do not fall under the scope of the present invention.

Formula I-F compounds are β-L-purine nucleosides having the structure: 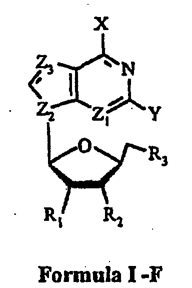 X is selected from H, R, -SNH₂, -S(O)NH₂, -SO₂NH₂, F, Cl, Br, I, N₃, -CN, -OR, -SR, -NR₂, where R is selected from H, alkyl, alkenyl, alkynyl, and aralkyl acetyl, acyl, sulfonyl;
Y is selected from H, R, F, Cl, Br, I, N₃, -CN, -OR, -SR, -NR₂, where R is selected from H, alkyl, alkenyl, alkynyl, and aralkyl, acetyl, acyl, sulfonyl;
Z₁, Z₂ and Z₃ are independently selected from C, N, and CH;
R₁, R₂, and R₃ are independently selected from H, -OR, -OAc, -OBz, -OP(O₂)OH.

### Uses

It is contemplated that compounds according to Formulas I-A, I-B , I-C, I-D, and I-E, the compounds of the present invention, will used to treat a wide variety of conditions, and in fact any condition which responds positively to administration of one or more of the compounds. Among other things it is specifically contemplated that compounds of the invention may be used to treat an infection, an infestation, a cancer or tumor or an autoimmune disease.

Infections contemplated to be treated with the compounds of the present invention include respiratory syncytial virus (RSV), hepatitis B virus (HBV), hepatitis C virus (HCV), herpes simplex type 1 and 2, herpes genitalis, herpes keratitis, herpes encephalitis, herpes zoster, human immunodeficiency virus (HIV), influenza A virus, hantann virus (hemorrhagic fever), human papilloma virus (HPV), measles and fungus.

Infestations contemplated to be treated with the compounds of the present invention include protozoan infestations, as well as helminth and other parasitic infestations.

Cancers or tumors contemplated to be treated include those caused by a virus, and the effect may involve inhibiting the transformation of virus-infected cells to a neoplastic state, inhibiting the spread of viruses from transformed cells to other normal cells and/or arresting the growth of virus-transformed cells.

Autoimmune and other diseases contemplated to be treated include arthritis, psoriasis, bowel disease, juvenile diabetes, lupus, multiple sclerosis, gout and gouty arthritis), rheumatoid arthritis, rejection of transplantation, allergy and asthma.

Still other contemplated uses of the compounds according to the present invention include use as intermediates in the chemical synthesis of other nucleoside or nucleotide analogs which are, in turn, useful as therapeutic agents or for other purposes.

In yet another aspect, a use of a compound according to the present invention for the manufacture of a pharmaceutical composition for treating a mammal comprises administering a therapeutically and/or prophylactically effective amount of a pharmaceutical containing a compound of the present invention. In this aspect the effect may relate to modulation of some. portion of the mammal's immune system, especially modulation of lymphokines profiles of Th1 and Th2. Where modulation of Th1 and Th2 lymphokines occurs, it is contemplated that the modulation may include stimulation of both Th1 and Th2, suppression of both Th1 and Th2, stimulation of either Th1 or Th2 and suppression of the other, or a bimodal modulation in which one effect on Thl/Th2 levels (such as generalized suppression) occurs at a low concentration, while another effect (such as stimulation of either Th1 or Th2 and suppression of the other) occurs at a higher concentration.

In general, the most preferred uses according to the present invention are those in which the active compounds are relatively less cytotoxic to the non-target host cells and relatively more active against the target. In this respect, it may also be advantageous that L-nucleosides may have increased stability over D-nucleosides, which could lead to better pharmacokinetics. This result may attain because L-nucleosides may not be recognized by enzymes, and therefore may have longer half-lives.

It is contemplated that compounds according to the present invention will be administered in any appropriate pharmaceutical formulation, and under any appropriate protocol. Thus, administration may take place orally, parenterally (including subcutaneous injections, intravenous, intramuscularly, by intrasternal injection or infusion techniques), by inhalation spray, or rectally, topically and so forth, and in dosage unit formulations containing conventional non-toxic pharmaceutically acceptable carriers, adjuvants and vehicles.

By way of example, it is contemplated that compounds according to the present invention can be formulated in admixture with a pharmaceutically acceptable carrier. For example, the compounds of the present invention can be administered orally as pharmacologically acceptable salts. Because the compounds of the present invention are mostly water soluble, they can be administered intravenously in physiological saline solution (e.g., buffered to a pH of about 7.2 to 7.5). Conventional buffers such as phosphates, bicarbonates or citrates can be used for this purpose. Of course, one of ordinary skill in the art may modify the formulations within the teachings of the specification to provide numerous formulations for a particular route of administration without rendering the compositions of the present invention unstable or compromising their therapeutic activity. In particular, the modification of the present compounds to render them more soluble in water or other vehicle, for example, may be easily accomplished by minor modifications (salt formulation, esterification, etc.) which are well within the ordinary skill in the art. It is also well within the ordinary skill of the art to modify the route of administration and dosage regimen of a particular compound in order to manage the pharmacokinetics of the present compounds for maximum beneficial effect in patients.

In certain pharmaceutical dosage forms, the pro-drug form of the compounds, especially including acylated (acetylated or other) derivatives, pyridine esters and various salt forms of the present compounds are preferred. One of ordinary skill in the art will recognize how to readily modify the present compounds to pro-drug forms to facilitate delivery of active compounds to a target site within the host organism or patient. One of ordinary skill in the art will also take advantage of favorable pharmacokinetic parameters of the pro-drug forms, where applicable, in delivering the present compounds to a targeted site within the host organism or patient to maximize the intended effect of the compound.

In addition, compounds according to the present invention may be administered alone or in combination with other agents for the treatment of the above infections or conditions. Combination therapies according to the present invention comprise, the administration of at least one compound of the present invention or a functional derivative thereof and at least one other pharmaceutically active ingredient. The active ingredient(s) and pharmaceutically active agents may be administered separately or together and when administered separately this may occur simultaneously of separately in any order. The amounts of the active ingredient(s) and pharmaceutically active agent(s) and the relative timings of administration will be selected in order to achieve the desired combined therapeutic effect. Preferably the combination therapy involves the administration of one compound of the Present invention or a physiologically functional derivative thereof and one of the agents mentioned herein below.

Examples of such further therapeutic agents include agents that are effective for the modulation of immune system or associated conditions such as AZT, 3TC, 8-substituted guanosine analogs, 2',3'-dideoxynucleosides, interleukin II, interferons such as □-interferon, tucaresol, levamisole, isoprinosine and cyclolignans. Certain compounds according to the present invention may be effective for enhancing the biological activity of certain agents according to the present invention by reducing the metabolism or inactivation of other compounds and as such, are co-administered for this intended effect.

With respect to dosage, one of ordinary skill in the art will recognize that a therapeutically effective amount will vary with the infection or condition to be treated, its severity, the treatment regimen to be employed, the pharmacokinetics of the agent used, as well as the patient (animal or human) treated. Effective dosages may range from 1 mg/kg of body weight, or less, to 25 mg/kg of body weight or more. In general a therapeutically effective amount of the present compound in dosage form usually ranges from slightly less than about 1 mg./kg. to about 25 mg./kg. of the patient, depending upon the compound used, the condition or infection treated and the route of administration. This dosage range generally produces effective blood level concentrations of active compound ranging from about 0.04 to about 100 micrograms/cc of blood in the patient. It is contemplated, however, that an appropriate regimen will be developed by administering a small amount, and then increasing the amount until either the side effects become unduly adverse, or the intended effect is achieved.

Administration of the active compound may range from continuous (intravenous drip) to several oral administrations per day (for example, Q.I.D.) and may include oral, topical, parenteral, intramuscular, intravenous, sub-cutaneous, transdermal (which may include a penetration enhancement agent), buccal and suppository administration, among other routes of administration.

To prepare the pharmaceutical compositions according to the present invention, a therapeutically effective amount of one or more of the compounds according to the present invention is preferably intimately admixed with a pharmaceutically acceptable carrier according to conventional pharmaceutical compounding techniques to produce a dose. A carrier may take a wide variety of forms depending on the form of preparation desired for administration, e.g., oral or parenteral. In preparing pharmaceutical compositions in oral dosage form, any of the usual pharmaceutical media may be used. Thus, for liquid oral preparations such as suspensions, elixirs and solutions, suitable carriers and additives including water, glycols, oils, alcohols, flavouring agents, preservatives, colouring agents and the like may be used. For solid oral preparations such as powders, tablets, capsules, and for solid preparations such as suppositories, suitable carriers and additives including starches, sugar carrier, such as dextrose, mannitol, lactose and related carriers, diluents, granulating agents, lubricants, binders, disintegrating agents and the like may be used. If desired, the tablets or capsules may be enteric-coated or sustained release by standard techniques.

For parenteral formulations, the carrier will usually comprise sterile water or aqueous sodium chloride solution, though other ingredients including those which aid dispersion may be included. Of course, where sterile water is to be used and maintained as sterile, the compositions and carriers must also be sterilized. Injectable suspensions may also be prepared, in which case appropriate liquid carriers, suspending agents and the like may be employed.

### Test Results

*In vitro* tests were performed on nine L-guanosine compounds were performed, and the results are described below. The nine compounds were as follows:
17316 8-mercapto-L-guanosine
17317 2-amino-9 β-L-ribofuransylpurine-6-sulfenamide ; comparative
17318 2-amino-9 β-L-ribofuransylpurine-6-sulfinamide ; comparative
17319 2-amino-9 β-L-ribofuransylpurine-6-sulfonamide ; comparative
17320 7-deaza-8-aza-β-L-guanosine
17321 7-deaza-8-aza-7-amino-β-L-guanosine
17322 7-deaza-8-aza-7-bromo-β-L-guanosine
17323 8-amino-1-βL-ribofuranosylthiazolo 4,3-dipyrimidine-2,7(3H,6H)-dione
17324 8-Allyloxy-β-L-guanosine

Peripheral blood mononuclear cells (PBMCs) were isolated from the buffy coat following Ficoll-Hypaque density gradient centrifugation of 60 ml blood from healthy donors. T-cells were then purified from the PBMCs using Lymphokwik lymphocyte isolation reagent specific for T-cells (LK-25T, One Lambda, Canoga Park CA). An average yield of 40 - 60 x 10⁶ T-cells were then incubated overnight at 37°C in 20 - 30 ml RPMI-AP5 (RPMI-1640 medium (ICN, Costa Mesa, CA) containing 20 mM HEPES buffer, pH 7.4, 5 % autologous plasma, 1 % L-glutamine, 1 % penicillin/streptomycin and 0.05 % 2-mercaptoethanol) to remove any contaminating adherent cells. In all experiments, T-cells were washed with RPMI-AP5 and then plated on 96-well microtitre plates at a cell concentration of 1 x 10⁶ cells/ml.

The T-cells were activated by the addition of 500 ng ionomycin and 10 ng phorbol 12-myristate 13-acetate (PMA) (Calbiochem, La Jolla, CA) and incubated for 48 - 72h at 37 °C. PMA/ionomycin-activated T-cells were treated with 0.5 - 50 µM of the L-guanosine being tested, or with 250 - 10000 U/ml of a control antiviral, interferon-alpha (Accurate, Westbury, NY) immediately following activation and re-treated 24 h later. T-cells from each plate were used for immunofluorescence analysis and the supernatants used for extracellular cytokine measurements. Following activation, 900 µl cell supernatant from each microplate was transferred to another microplate for analysis of cell-derived cytokine production. The cells are then used in immunofluorescence analyses for intracellular cytokine levels and cytokine receptor expression.

Cell-derived human cytokine concentrations were determined in cell supernatants from each microplate. Activation-induced changes in interleukin-2 (IL-2) levels were determined using a commercially available ELISA kit (R & D systems Quantikine kit, Minneapolis, MN) or by bioassay using the IL-2-dependent cell line, CTLL-2 (ATCC, Rockville, MD). Activation -induced changes in interleukin-4 (IL-4), tumor necrosis factor (TNFα) interleukin-8 (IL-8) (R & D systems (Quantikine kit, Minneapolis, MN) and interferon-gamma (IFN-γ) (Endogen (Cambridge, MA) levels were determined using ELISA kits. All ELISA results were expressed as pg/ml and the CTLL-2 bioassay as counts per minute representing the IL-2-dependent cellular incorporation of ³H-thymidine (ICN, Costa Mesa, CA) by CTLL-2 cells.

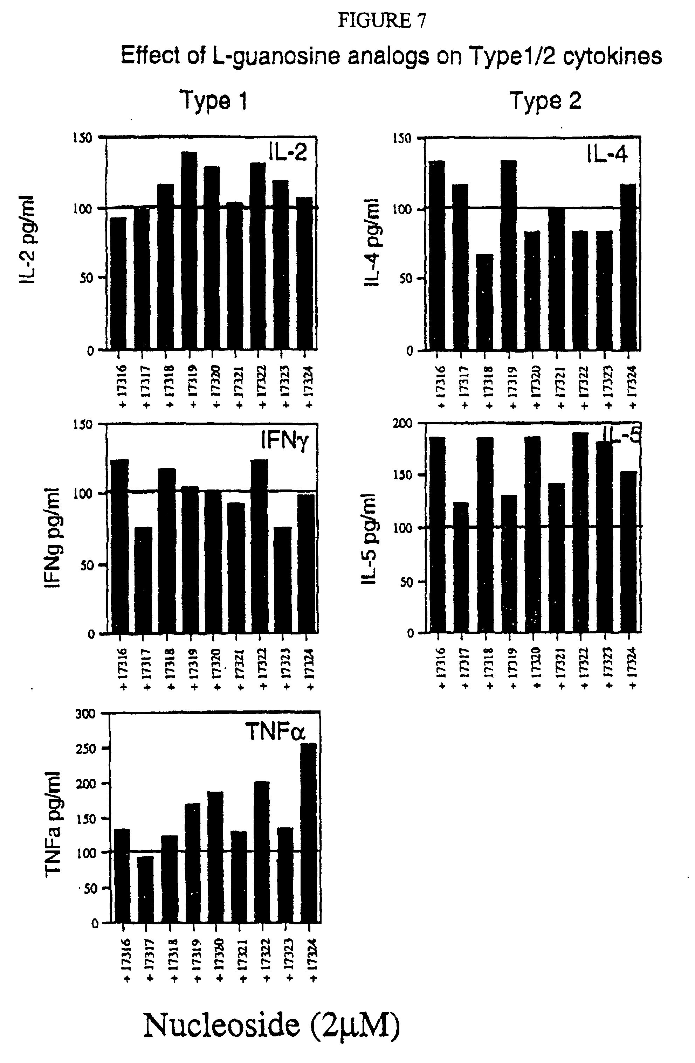

The results for each of the nine L-guanosine analogs on IL-2 TNFα, IFN-γ, IL-4 and IL-5 levels are presented in Figures7.

### Synthesis

The compounds of the present invention may be produced according to synthetic methods which are individually readily known to those of ordinary skill in the art. In general, compounds according to the present invention are synthesized by condensing appropriate nucleoside base with the necessary sugar synthon to give the protected L-nucleoside which on further manipulation and deprotection of the sugar hydroxyl protecting groups will ultimately give rise to nucleoside analog having the desired ribofuranosyl moiety of the L-configuration.

Scheme 1 shows the synthesis of certain 7- and 8-substituted L-guanosine analogs. L-ribose **1** was methylated at C-1 and the resulting product 2 benzoylated to give compound **3**, which was converted to **4** by treatment with acetic anhydride and in the presence of sulfuric acid. Reaction of **4** and silylated N²-acetyl guanine in the presence of trimethylsilyl triflate gave compound **5** according to a commonly used procedure (Vorbruggen et al. *Chem. Ber.,* **1981,** 14, 1234). **5** was converted to **6** with ammonia in methanol. Bromination of **5** gave 8-bromo derivative **7**, which was converted to 8-allyloxy-derivative **8** by treatment with allyl alcohol and sodium hydride. **8** was heated in water-methanol to yield 7-allyl-8-oxo derivative **9,** which was hydrogenated to give 7-propyl-8-oxo-L-guanosine **10.**

Scheme 2 shows the synthesis of N²-acetyl-3-deaza-L-guanosine. 3-Deazaguanine **11** (Cook et al. *J. Med. Chem.* **1976,** *27*, 1389) was treated with acetic anhydride in pyridine to yield N²-acetyl-3-deazaguanine **12,** which was silylated and coupled with 1-acetyl-2,3,5-*O*-tribenzoyl-L-ribose to give compound **13.** Removal of benzoyl group with ammonia-methanol yielded N²-acetyl-3-deaza-L-guanosine **14.**

Scheme 3 shows the synthesis of 6-mercapto-L-guanosine and derivatives. N²-Acetyl-2',3',5'-*O*-tribenzoyl-β-L-guanosine **5** was converted by treatment with phosphoruspentasulfide (Fox, et al. *J. Am. Chem. Soc.* **1958,** *80*, 1669) to 6-mercapto derivative **15,** which was deprotected to give 6-mercapto-β-L-guanosine **16.** The sulfenamide derivative **17** was prepared by reaction of **16** with NH₂-Cl generated in situ. The sulfenamide **17** was oxidized with MCPBA to the sulfinamide **18** and sulfonamide **19** by controlling amount of the reagent (Revankar et al. *J. Med. Chem.* **1990,** *33*, 121).

Scheme 4 shows the synthesis of 1-β-L-ribofuranosylpyranzolo[3,4-d]pyrimidin-4(5H)-one and derivatives. The commercially available 4-hydroxypyranzolo[3,4-d]pyrimidine **20** was coupled with protected L-ribose to give the protected nucleoside **21,** which was deprotected to give 1-β-L-ribofuranosylpyranzolo[3,4-d]pyrimidin-4(5H)-one **22.** Similarly, 3-bromo-4-hydroxypyranzolo[3,4-d]pyrimidine **23** (Cottam et al. *J. Med. Chem..* **1984,** *27*, 1119) was coupled with L-ribose to give the protected nucleoside **24,** which was deprotected to give 3-bromo-1-β-L-ribofuranosylpyranzolo[3,4-d]pyrimidin-4(5H)-one **25** Treatment of **24** with ammonia in the presence of copper and cuprous chloride at 100 °C yielded the 3-amino derivative **26.**

Scheme 5 shows the synthesis of 8-aza-7-deaza-L-guanosine analogs. 3,6-Dibromopyrazolo[3,4-d]pyrimidin-4(5H)-one **27** (Petrie III et al. *J*. *Med. Chem.* **1985,** *28,* 1010) was coupled with protected L-ribose to give the nucleoside **28,** which was treated with ammonia to give 8-aza-3-bromo-7-deaza-β-L-guanosine **29.** Treatment of **28** with ammonia at 120°C yielded 3-amino derivative **30.** Hydrogenation of **29** over Pd/C gave 8-aza-7-deaza-β-L-guanosine **31.**

Scheme 6 shows the synthesis of 5-amino-3-β-L-ribofuranosylthiazolo[4,5-d]pyrimidine-2,7(6H)-dione and analogs. 5-Aminothiazolo[4,5-d]pyfimidine-2,7(3H, 6H)-dione **32** (Baker et al. *J. Chem. Soc. C* **1970,** 2478) was coupled with the deprotected ribose to give the nucleoside **33,** which was deprotected to give 5-amino-3-β-L-ribofuranosylthiazolo[4,5-d]pyrimidine-2,7(6H)-dione **34.** Compound **33** can be protected with nitrophenethyl group and then treated with butyl nitrite and hydrogen fluoride in pyridine to give the fluoride derivative **35.** Treatment of **33** with t-butyl nitrite (Nagahara et al. *J. Med*. *Chem.* **1990,** *33*, 407) in THF can replace the amino group with hydrogen to give **36.**

Scheme 7 shows the synthesis of 3-deaza-L-guanosine and derivative. The imidazole derivative **37** was silylated and reacted with **4** to give **38,** which yielded **39** through cyclisation. Bromination of **39** afforded **40.**

The compounds described in schemes 1-6 are β-L-guanosine analogs. The corresponding α-L-analogs can be prepare in the similar manner, but with L-ribose having different protecting groups. 1-Acetyl-2,3,5-*O*-tribenzoyl-L-ribofuranose can be replaced with 1-bromo-β-L-ribose derivatives as reagent, which would produce α-L-nucleosides as major products.

### Examples

Examples 1-5, 9-16, 26, 28-30 are merely preparative or comparative examples and do not fall under the scope of the present invention

### Example 1

### 1-O-Methyl-L-ribofuranose 2

A cold solution of dry hydrogen chloride (4.4 g, 0.12 mol) in methanol (100 mL) was slowly added to the solution of L-(+)-ribose 1 (50 g, 0.33 mole in methanol (1000 mL) at room temperature After addition, the solution was stirred for 2.5 h and quenched with pyridine (100 mL). The mixture was stirred for 10 min and the solvent was evaporated. The residue was dissolved in pyridine (100 mL) and the resulting solution was concentrated to dryness to give 1-*O*-methyl-L-ribofuranose **2** as a pale-yellow syrup

### Example 2

### 1-O-Methyl-2',3',5'-O-tribenzoyl-L-ribofuranose 3

Benzoyl chloride (154.5 g, 1.1 mol) was added dropwise during 10 min to a solution of 1-*O*-methyl-L-ribofuranose **2** (0.33 mol) in pyridine (350 mL) at 0°C. After addition, the solution stood at room temperature for 14 h and quenched by stirring with water (50 mL) at 0 oC for 1 h. The aqueous layer was extracted with CH₂Cl₂ (2 x 100 mL) and the combined organic layer concentrated. The residue was dissolved in CH₂Cl₂ (500 mL), washed successively with saturated NaHCO₃ (3 x 100 mL), water (200 mL), brine (200 mL), dried over Na2SO4, filtered, and evaporated with toluene (2 x 300 mL). Further drying under vacuum afforded 1-*O*-methyl-2',3',5'-O-tribenzoyl-L-ribofuranose **3** as a yellow syrup (80 g, 0.17 mole).

### Example 3

### 1-O-Acetyl-2',3',5'-O-tribenzoyl-L-ribofuranose 4

1-O-Methyl-2',3',5'-O-tribenzoyl-L-ribofuranose **3** (80 g, 0.17 mol) was dissolved at room temperature in a mixture of acetic acid (354 mL) and acetic anhydride (36 mL). The resulting solution was cooled to 0 oC and sulfuric acid (96%, 8.23 g, 0.084 mol) added dropwise. After addition, the reaction mixture stood at room temperature for 18 h, poured onto ice (500 g), and stirred until the ice had melt. EtOAc (1.2 L) was added followed by water (1L). The organic layer was washed with water/brine mixture (4/1 ratio), saturated NaHCO3 (500 mL), brine (500 mL), filtered through a silica gel pad, and concentrated to give the crude product as yellow solid. Recrystallization from hexanes/EtOAc (300 mL/100 mL ratio) afforded 1-*O*-acetyl-2',3',5'-*O*-tribenzoyl-L-ribofuranose **4** as white needles (50 g, 59.6% overall yield from L-ribose).

### Example 4

### N²-Acetyl-2',3',5'-O-tribenzoyl-β-L-guanosine 5

N²-Acetylguanine (4.125g, 21.35mmol) was suspended in pyridine (50 mL) at 80°C for 25 min. and then pyridine was evaporated under high vacuum. The same procedure was repeated once. The obtained material was dried under vacuum overnight and silylated by heating with excess ofHMDS (50mL), pyridine (10mL) and TMSCI (150 □L) under argon for 2.5 hours. After the reaction mixture was cooled to RT, the solvents were evaporated under vacuum. The residual HMDS and pyridine were coevaporated with xylene (2 x 40mL). The silylated base was suspended in dichloroethane (70 mL) and combined with dichloroethane (182mL) solution of 1-acetyl-2,3,5-*O*-tribezoyl-L-ribose (9.71g, 19.22 mmol). The obtained suspension was stirred under argon at reflux temperature for 10 min. and dichloroethane (35 mL) solution of TMS-triflate (4.50 mL, 23.276 mmol) was added dropwise (20 min.). The obtained reaction mixture was stirred under reflux for 1.5 h, cooled to RT and diluted with methylene chloride (500ml). The organic solution was washed with cold NaHCO₃ (5% aq., 2 x 150mL), brine (150 mL), dried (Na₂SO₄) and evaporated to dryness. The reaction mixture was purified by flash chromatography (400 g of silica gel, eluent: 28% EtOAc, 2% EtOH in CH₂Cl₂, v/v) to give 5.60g (46%) of N²-Acetyl-2',3',5'-*O*-tribenzoyl-β-L-guanosine **5.**

### Example 5

### β-L-Guanosine 6

A solution of N²-acetyl-2',3,5'-*O*-tribenzoyl-L-guanosine **5** in saturated ammonia-methanol stood at room temperature for two days. Ammonia and methanol were evaporated and the crude dissolved in water and chloroform (two layers). The aqueous layer was washed with chloroform three times and concentrated. The crude product was purified by crystallization from water-methanol to give β-L-Guanosine **6** as a colorless solid.

### Example 6

### 8-Bromo-β-L-guanosine 7

To a suspension of L-guanosine **6** (1.24 g) in water (7.5 mL) was added in portions 35 mL of saturated bromine-water containing 0.35 mL of bromine. The solid was filtered off, successively washed with cold water, cold acetone, and dried. Crystallization from water gave pure 8-Bromo-L-guanosine **7** as a colorless solid.

### Example 7

### 8-Allyloxy-β-L-guanosine 8

To a stirred mixture of NaH (984 mg) in anhydrous DMSO (30 mL) was added dropwise allyl alcohol (10 mL), followed by addition of 8-Bromo-L-guanosine 7 (1.78 g, 4.92 mmol) in DMSO (10 mL). The resulting reaction mixture was stirred at 60 oC overnight, cooled to room temperature, and diluted with ethyl ether (350 mL). The resulting precipitates were filtered, dissolved in water (18 mL), and neutralized with acetic acid. The resulting precipitates were filtered and recyrstallized from water/methanol to give 836 mg of 8-allyloxy-L-guanosine **8** as a slightly yellow solid.

### Example 8

### 7-Allyl-8-oxo-β-L-guanosine 9

A mixture of 8-allyloxyguanosine **8** (560 mg) in methanol-water (50 mL, 1:1, v/v) was stirred under reflux and a clear solution formed after two hours. The solution was refluxed for additional 5 h and cooled to room temperature. A brown precipitates (by product) was filtered and the filtrate concentrated to give a crude product. Crystallization from water-ethanol gave 83 mg of title compound as a slightly brown solid. The filtrate was concentrated and the residue chromatographed on silica with 5%Et3N and 20%MeOH in methylene chloride to give 260 mg of 7-allyl-8-oxo-β-L-guanosine **9** as a colorless solid.

### Example 9

### 8-Oxo-7-propyl-β-L-guanosine 10

A suspension of 120 mg of 7-allyl-8-oxo-b-L-guanosine **9** and 80 mg of 10% palladium on carbon was shaken in a hydrogenation apparatus at room temperature under 55 psi hydrogen for 2 h. Palladium catalyst was filtered and the filtrate concentrated. The crude was crystallized from water-ethanol to give 75 mg of 8-Oxo-7-propyl-β-L-guanosine **10** as a slightly yellow solid.

### Example 10

### N²-Acetyl-3-deazaguanine 12

To a suspension of 3-Deazaguanine **11** (2.0 g) in anhydrous pyridine (30 mL) was added acetic anhydride (5 mL) and the resulting reaction mixture heated to 90 oC. Solid was dissolved gradually and a brown solution formed. After 10 minutes the precipitates reoccurred. The mixture was stirred at 90 oC for additional 90 minutes and cooled to 50 oC. The precipitates were filtered and washed with acetonitrile, water, and acetonitrile again to give 1.79 g of N²-acetyl-3-deazaguanine **12** as a light-brown solid.

### Example 11

### N²-Acetyl-3-deaza-β-L-guanosine 14

A suspension of N²-acetyl-3-deazaguanine **12** (576 mg, 3.0 mmol), hexamethyldisilazane (HMDS, 15 mL), pyridine (2mL), and ammonium sulfate (10 mg) was stirred under reflux and exclusion of moisture for 2.5 h. Solvents were evaporated and the residue dried under vacuum for 2 h to give a foam syrup. The residue was dissolved in methylene chloride (anhydrous, 30 mL) and 1-Acetyl-2,3,5-tribenzoyl-L-robose (1.51 g, 3.0 mmol) added, followed by slow addition of trimethylsilyl triflate (4.5 mmol, 0.81 mL). The resulting solution was refluxed for 20 h. Solvent was evaporated and the residue dissolved in ethyl acetate, washed with 5% NaHCO3, dried (NA2SO4), and concentrated. Chromatography on silica with 5%Et3N and 2-10% ethanol in methylene chloride gave three major products: 340 mg of higher Rf product, 368 mg of the medium Rf product, and 335 mg of the lower Rf product, all as a slightly yellow solid.

A solution of the medium Rf product **13** (350 mg) in saturated ammonia-methanol stood at room temperature for two days. Ammonia and methanol were evaporated and the residue was chromatographed on silica with 5%Et3N and 20% ethanol in methylene chloride to give 114 mg of N²-acetyl-3-deaza-β-L-guanosine as **14** as a white solid.

### Example 12

### N²-Acetyl-6-mercapto-2',3',5'-O-tribenzoyl-β-L-guanosine 15

To a stirred suspension of N²-acetyl-2',3',5'-*O*-tribenzoyl-L-guanosine **5** (5.60g, 8.78 mmol) and phosphoruspentasulfide (8.0g, 36.0mmol) in pyridine (210 mL) was added dropwise water (590 □L) and the resulting reaction mixture heated at reflux temperature for 8 h. A few drops of water was added whenever the solution began to lose its turbidity. At the end of reflux period pyridine was evaporated to give a thin syrup, which was added slowly to vigorously stirred, boiling water (1000 mL). The resulting mixture was stirred for 45 minutes and extracted with EtOAc (3 x 250 mL). The organic layer was washed with brine (2 x 200mL), water (2 x 100mL), dried (Na₂SO₄), and evaporated to dryness. Chromatography on silica gel (400g) with 23% EtOAc, 2% EtOH in CH₂Cl₂ (v / v) to give 3.53g (61.5%) of N²-acetyl-6-mercapto-2',3',5'-*O*-tribenzoyl-β-L-guanosine **15** as a colorless solid.

### Example 13

### 6-mercapto-β-L-guanosine 16

A solution of N²-acetyl-6-mercapto-2',3',5'-*O*-tribenzoyl-L-guanosine **15** (3.53 g, 5.40 mmol) in saturated ammonia-methanol (200mL) was stirred at room temperature for 62 hours. Ammonia and methanol were evaporated and the residue was triturated with chloroform. The precipitates were filtered and washed with warm chloroform (50mL), redissolved in dilute aqueous ammonia, and acidified with acetic acid. The resulting precipitates were filtered and dried under vacuum to give 1.48 g (91.6%) of 6-mercapto-β-L-guanosine **16** as a colorless solid.

### Example 14

### 2-amino-9-(β-L-ribofuranosyl)purine-6-sulfenamide 17

To a stirred, aqueous sodium hypochlorite solution (5.25%, 2.25mL, 1.725mmol) cooled to 0 °C in an ice bath was added ammonium hydroxide (1.4 M, 6 mL, 8.4 mmol) cooled to 0°C. The resulting mixture was stirred at 0 °C for 15 minutes and a cold (0 °C) solution of 6-mercapto-L-guanosine **16** (450 mg, 1.5 mmol) in 2M KOH (750 mL) was added. The reaction mixture was stirred for 2 h until it had warmed to room temperature. The resulting precipitates were filtered off, washed with cold EtOH, filtered, and dried to give 240mg (51%) of 2-amino-9-(β-L-ribofuranosylpurine-6-sulfenamide **17** as a colorless solid.

### Example 15

### 2-Amino-9-(β-L-ribofuranosyl)purine-6-sulfinamide 18

A mixture of 2-amino-9-(β-L-ribofuranosyl)purine-6-sulfenamide **17** (200mg, 0.637 mmol), ethanol (90 mL) and water (6.4mL) was vigorously stirred at -10 oC in a salt-ice bath. A solution of MCPBA (80%, 137.0 mg, 0.637mmol) in ethanol 5.5mL was added dropwise over a period of 15 minutes. The mixture was allowed to stir and warm as the ice melt (8 h), and stirred at ambient temperature for addition 14 h. A small amount of precipitate was filtered out and the filtrate evaporated at 23 oC to dryness. The residue was triturated with ethyl ether (30mL) and the solid was collected by filtration, washed with ethyl ether (10 mL). The solid was again suspended in ethyl ether (25 mL), filtered, and dried to give 182mg (87%) of 2-amino-9-(β-L-ribofuranosyl)purine-6-sulfinamide **18** as a colorless solid.

### Example 16

### 2-Amino-9-(β-L-ribofuranosyl)purine-6-sulfonamide 19

To a stirred suspension of 2-amino-9-(β-L-ribofuranosyl)purine-6-sulfenamide **17** (150 mg, 0.478 mmol) in ethanol (28.5mL) and water (2.8mL) at room temperature was added in portions during 1 hour a solution of MCPBA (80%, 412.0 mg, 1.91mmol) in ethanol (2.8 mL). The reaction mixture became clear after 3 h. The solution was stirred for an additional 15 h at ambient temperature and became cloudy. The reaction mixture was concentrated at room temperature to dryness. The residue was triturated with ethyl ether (30 mL) and the solid was collected by filtration. The crude product was dissolved in methanol/water mixture and adsorbed onto silica gel (2.0 g). Solvent was evaporated and the dry silica bearing the product was loaded onto a flash silica column (100 g) packed in methylene chloride. The column was eluted with 20% MeOH in CH₂Cl₂ (v / v)to give 87mg (52.6%) of 2-Amino-9-(β-L-ribofuranosyl)purine-6-sulfonamide **19** as a colorless solid.

### Example 17

### 1-(2',3',5'-O-tribenzoyl-β-L-ribofuranosyl)pyrazolo[3,4-d]pyrimidin 4(5H)-one 21

A mixture of 4-Hydroxypyrazolo[3,4-d]pyrimidine 20 (100 mg, 0.74 mmole), 1,1,1,3,3,3-hexamethyldisilazane (HNDS, 10 mL), and (NH₄)₂SO₄ (10 mg, 0.076 mmole) was heated under reflux for 3 h to form a clear solution. The excess HMDS was evaporated to give a yellow solid, which was dried under vacuum for 15 min. 1-*O*-Acetyl-2',3',5'-*O*-tribenzoyl-L-ribofuranose (370 mg, 0.74 mmol) was added, followed by addition of acetonitrile (anhydrous, 5 mL). Trimethylsilyl trifluoromethanesulfonate (245 mg, 1.1 mmol) was added dropwise to the above slurry at room temperature. After addition, the clear solution stood at room temperature for 14 h. Solvent was evaporated and the yellow residue dissolved in EtOAc (50 mL), washed with saturate NaHCO₃ (2 x 20 mL), water (3 x 20 mL), dried over Na₂SO₄, and concentrated. Flash chromatography on silica (5% methanol in methylene chloride) gave 1-(2',3',5'-*O*-tribenzoyl-β-L-ribofuranosyl)pyrazolo[3,4-d]pyrimidin-4(5H)-one **21** as a white solid (177 mg, 41.5%).

### Example 18

### 1-β-L-ribofuranosylpyrazolo[3,4-d]pyrimidin-4(5H)-one 22

1-(2',3',5'-*O*-Tribenzoyl-β-L-ribofuranosyl)pyrazolo[3,4-d]pyrimidin-4(5H)-one **21** (152 mg, 0.26 mmole) was dissolved in MeOH saturated with NH₃ at 0 □C (75 mL). The resulting solution stood at room temperature for 24 h and concentrated. The residue was dissolved in water (30 mL), washed with EtOAc (3 x 15 mL). After evaporation of the water, the crystalline solid was soaked in acetonitrile (2 mL), filtered, and dried under vacuum to give 1-β-L-ribofuranosylpyrazolo[3,4-d]pyrimidin-4(5H)-one **22** as a white crystalline solid (70 mg, 99%).

### Example 19

### 3-Bromo-1-(2',3',5'-O-tribenzoyl-β-L-ribofuranosyl)pyrazolo[3,4-d]pyrimidin-4(5H)-one 24

Acetonitrile (30 mL) was added to a mixture of 3-bromopyrazolo[3,4-d]pyrimidin-4(5H)-one **23** (1.08 g, 4.0 mmoles) and 1-*O*-acetyl-2',3',5'-*O*-tribenzoyl-β-L-ribofuranose (3.02 g, 6.0 mmoles). The resulting slurry was heated to reflux and trifluoroborane etherate (851 mg, 6.0 mmoles) added dropwise. The resulting solution was heated under reflux overnight. Solvent was evaporated, residue dissolved in EtOAc (100 mL), the resulting solution washed with saturated NaHCO₃, water, dried over Na₂SO₄, and concentrated. Flash chromatography on silica (5% acetone in methylene chloride) afforded 3-Bromo-1-(2',3',5'-O-tribenzoyl-β-L-ribofuranosyl)pyrazolo[3,4-d]pyrimidin-4(5H)-one **24** as a pale-yellow solid (1.1 g, 41.7%).

### Example 20

### 3-Bromo-1-β-L-ribofuranosylpyrazolo[3,4-d]pyrimidin-4(5H)-one 25

3-Bromo-1-(2',3',5'-*O*-tribenzoyl-β-L-ribofuranosyl)pyrazolo[3,4-d]pyrimidin-4(5H)-one **24** (280 mg, 0.43 mmole) was dissolved in MeOH saturated with NH₃ at 0 □C (25 mL). The solution in a sealed, stainless steel bomb was heated at 100 □C for 6 h. After cooling, ammonia and methanol were evaporated. The residue was dissolved in water (40 mL), washed with EtOAc (4 x 20 mL), and concentrated. The residue was soaked in acetonitrile and the resulting solid filtered, dried under vacuum to give 3-Bromo-1-β-L-ribofuranosylpyrazolo[3,4-d]pyrimidin-4(5H)-one **25** as a white solid (140 mg, 95%).

### Example 21

### 3-Amino-1-β-L-ribofuranosylpyrazolo[3,4-d]pyrimidin-4(5H)-one 26

3-Bromo-1-(2',3',S'-*O*-tribenzoyl-β-L-ribofuranosyl)pyrazolo[3,4-d]pyrimidin-4(5H)-one **24** (714 mg, 1.08 mmoles) was dissolved in MeOH saturated with NH₃ at 0 □C (30 mL). Thin copper wire (21 mg, 0.33 mmole) and cuprous chloride (33 mg, 0.33 mmole) were added. The mixture in a sealed, stainless steel bomb was heated at 100 □C for 16 h. After cooling, silica gel (2 g) was added to the reaction mixture and the solvent evaporated. The silica gel absorbed with the crude product was loaded onto a silica column and eluted with 5% Et₃N, 17% MeOH in CH₂Cl₂). The product was further purified by recrystallization (95% EtOH) to afford 3-amino-1-β-L-ribofuranosylpyrazolo[3,4-d]pyrimidin-4(H)-one **26** as a white needles (110 mg, 36%).

### Example 22

### 3,6-Dibromo-1-(2',3',5'-O-tribenzoyl-β-L-ribofuranosyl)pyrazolo[3,4-d]pyrimidin-4(5H)-one 28

Acetonitrile (80 mL) was added to a mixture of 3,6-Dibromopyrazolo[3,4-d]pyrimidin-4(5H)-one **27** (1.18 g, 4.0 mmol) and 1-*O*-acetyl-2',3',5'-*O*-trihenzoyl-L-ribofuranose (3.02 g, 6.0 mmol). The slurry was heated to reflux, followed by slow addition of trifluoroborane etherate (851 mg, 6.0 mmol). The reaction mixture was heated under reflux for 6 h. After removal of solvent, the residue was dissolved in EtOAc (200 mL), washed with saturated NaHCO₃ (2 x 50 mL), water (2 x 50 mL), dried (Na₂SO₄), and concentrated. The crude product was purified by flash chromatography on silica (5% acetone in methylene chloride) to give (1.49 g, 50.5%) of 3,6-Dibromo-1-(2',3',5'-*O*-tribenzoyl-β-L-ribofuranosyl) pyrazolo[3,4-d]pyrimidin-4(5H)-one **28** as a yellow foam.

### Example 23

### 3-Bromo-7-deaza-8-aza-β-L-guanosine 24

3,6-Dibromo-1-(2',3',5'-*O*-tribenzoyl-b-L-ribofuranosyl)pyrazolo[3,4-d]pyrimidin-4(5H)-one (260 mg, 0.35 mmole) **28** was dissolved in MeOH saturated with NH₃ at 0 □C (20 mL). The solution in a sealed, stainless steel bomb was heated at 120 □C for 16 h. After cooling and removal of solvent, the residue was dissolved in water (100 mL), washed with CH₂Cl₂ (5 x 1 mL), and concentrated to give a yellow solid. The solid was dissolved in a mixture of methanol and methylene chloride (1:1) and passed through a silica gel pad The filtrate was concentrated and the solid residue dissolved in MeOH (5 mL), followed by slow addition of diethyl ether (40 mL). The resulting precipitates were filtered, washed with diethyl ether (2 x 2 mL), and dried under vacuum to give 3-bromo-7-deaza-8-aza-β-L-guanosine **29** as an off-white solid (102.2 mg, 80.2%).

### Example 24

### 3-Amino-7-deaza-8-aza-L-guanosine 25

3,6-Dibromo-1-(2',3',5'-*O*-tribenzoyl-β-L-ribofuranosyl)pyrazolo[3,4-d]pyrimidin-4(5H)-one **28** (500 mg, 0.68 mmole) was dissolved in MeOH saturated with NH₃ at 0 □C (50 mL), followed by addition of thin copper wire (21.5 mg, 0.34 mmole) and cuprous chloride (19.8 mg, 0.20 mmole). The mixture in a sealed, stainless steel bomb was heated at 120 □C for 16 h. After cooling and removal of solvent, the residue was dissolved in MeOH, the solid filtered, and the filtrate concentrated. Purification of the residue by flash chromatography on silica (20% MeOH in CH₂Cl₂) gave 3-amino-7-deaza-8-aza-L-guanosine **30** as a white solid (62 mg, 30.9%).

### Example 25

### 7-Deaza-8-aza-L-guanosine 26

3-Bromo-7-deaza-8-aza-β-L-guanosine **29** (246 mg, 0.68 mmole) was dissolved in EtOH (50%, 60 mL), followed by addition of 10% Pd/C (67 mg). The mixture was shaken at 50 psi hydrogen at room temperature for 6 h. The Palladium catalyst was filtered and the filtrate concentrated. The crude product was dissolved in MeOH, followed by addition of silica gel (2 g). After removal of methanol, the dry silica gel adsorbed with the crude product was loaded onto a silica column and eluted with 17% MeOH in CH2Cl2) to give 7-Deaza-8-aza-β-L-guanosine **31** as a white solid (102.4 mg, 53.2%).

### Example 26

### 5-Amino-3-β-L-ribofuranosylthiazolo[4,5-d]pyrimidine-2,7(6H)-dione 34

5-Aminothiazolo[4,5-d]pyrimidine-2,7(6H)-dione 32 (400 mg, 2.71 mmol) was suspended in acetonitrile (16 mL) and hexamethyldisilazane (0.96 mL), and trimethylchlorosilane (0.55 mL) and trimethylsilyl triflate (0.9 mL) added. The mixture was stirred under reflux for 3.5 h. A solution of trimethylsilyl triflate (0.45 mL) in acetonitrile (1.0 mL) was added dropwise and stirring and heating was continued for additional 30 min. A slurry of 1-O-acetyl-2,3,5-O-tribenzoyl-L-ribofuranose (1.22 g, 2.28 mmol) in acetonitrile (4.1 mL) was added and the mixture stirred under reflux for 30 min. The reaction mixture was cooled and slowly poured into a vigorously stirred mixture of sodium bicarbonate (2.81 g)and water (96 mL), which produced a stick solid. Ethyl acetate was added, the mixture stirred until the solid dissolved. The aqueous layer was extracted with ethyl acetate twice and the combined organic layer washed with sodium bicarbonate, dried (Na2SO4), and concentrated. The crude was purified by chromatography on silica with 5% Et3N and 5% ethanol in methylene chloride to give 1.10 g of 5-amino-3-(2',3',5'-*O*-tribenzoyl-β-L-ribofuranosyl)thiazolo[4,5-d]pyrimidine-2,7(6H)-dione **33** as a white solid.

5-Amino-3-(2',3',5'-*O*-tribenzoyl-β-L-ribofuranosyl)thiazolo[4,5-d]pyrimidine-2,7(6H)-dione **33** (1.09 g, 1.717 mmol) was dissolved in methanol (25 mL) and sodium methoxide (5.4 M in methanol, 0.64 mL) added. The solution stood at room temperature for 64 h. Most methanol was evaporated and water (20 mL) and Amberite H-Form (1,0 g) added. The suspension was stirred gently for 20 min and the resin filtered by suction, washed with water (2 x 10 mL). The filtrate was concentrated and the crude product was purified by crystallization from methanol to give 368 mg of 5-Amino-3-β-L-ribofuranosylthiazolo[4,5-d]pyrimidine-2,7(6H)-dione **34** as a colorless solid.

### Example 27

### 3-β-L-Ribofuranosylthiazolo[4,5-d]pyrimidine-2,7(6H)-dione 36

To a solution of 5-amino-3-β-L-ribofuranosylthiazolo[4,5-d]pyrimidine-2,7(6H)-dione **34** (1.40 g, 2.22 mmol) in anhydrous THF (50 mL) at r.t. was added dropwise t-butyl nitrite (15.05 mmol, 1.72 mL). The resulting solution was stirred at r.t. for 1 h and at 50 °C for 14 h. Solvent was evaporated and the residue chromatographed on silica with 20-30% ethyl acetate in methylene chloride to give 612 mg of deaminated product as a foam.

500 mg of the deaminated product was dissolved methanol (15 mL) and 30% ammonium hydroxide (75 mL) added. The resulting solution stood at r.t. overnight. Solvent was evaporated and the residue chromatographed on silica with 10-20% methanol in methylene chlorides to give 184 mg of 3-β-L-ribofuranosylthiazolo[4,5-d]pyrimidine-2,7(6H)-dione **36** as a colorless solid.

### Example 28

### Methyl 5-cyanomethyl-1-(2,3,5-O-tribenzoyl-L-ribofuranosyl)imidazole-4-carboxylate 38

Methyl 5-cyanomethylimidazole-4-carboxylate **37** (Robins et al. *J Org. Chem.* **1963,** *28*, 3041, 500 mg, 3.02 mmol) was refluxed under anhydrous conditions for 12 h with HMDS (8 mL) and ammonium sulfate (30 mg). The excess HMDS was removed by distillation under reduced pressure to give the trimethylsilyl derivative as a yellowish brown oil. The oil was dissolved in dry 1,2-dichloroethane (20 mL) and 1-O-acetyl-2,3,5-O-tribenzoylribofuranose (1.53 g, 3.03 mmol) was added, followed by addition of stannic chloride (516 □L, 4.39 mmol). The reaction mixture was stirred at ambient temperature for 18 h and then poured into a cold 5% NaHCO₃ aqueous solution (50 mL). The precipitates were filtered through Celite and the filtrate extracted with chloroform (3 x 50 mL). The extracts were dried (Na₂SO₄) and evaporated under reduced pressure to give a light -beige foam (1.8 g). This material was purified by chromatography on silica with hexanes-ethylacetate (1:1) to yield 1.65 g (89%) of Methyl 5-cyanomethyl-1-(2,3,5-O-tribenzoyl-L-ribofuranosyl)imidazole-4-carboxylate **38** as a colorless solid.

### Example 29

### 3-Deaza-β-L-guanosine 39

Methyl 5-cyanomethyl-1-(2,3,5-O-tribenzoyl-L-ribofuranosyl)imidazole-4-carboxylate **38** (1.03 g, 1.69 mmol) was dissolved in methanol (60 mL) and saturated with anhydrous ammonia at 0 °C. The reaction mixture was placed in a sealed steel bomb and kept at 100 °C for 18 h. The mixture was cooled to r.t. and evaporated to dryness. The residue was suspended in warm chloroform, and remaining solid was filtered, washed with chloroform (5 x 10 ml), and dried. The crude product was recyrstallized from water to yield 320 mg (70%) of 3-Deaza-β-L-guanosine **39** as a colorless solid.

### Example 30

### 3-Bromo-3-deaza-β-L-guanosine 40

To a stirred solution of 3-deaza-β-L-guanosine **39** (200 mg, 0.708 mmol) in 8 mL of water/methanol (1:1) at 0 °C was added bromine (20 □L, 0.39 mmol) was added. After stirring for 15 min. the reaction mixture was evaporated to dryness. The crude material was suspended in chloroform, filtered, and dried to yield 210 mg (82%) of 3-bromo-3-deaza-β-L-guanosine **40** as a colorless solid.

## Claims

1. A compound, an 8-substituted α-or β-L-guanosine analog, having a structure according to Formula I-A, 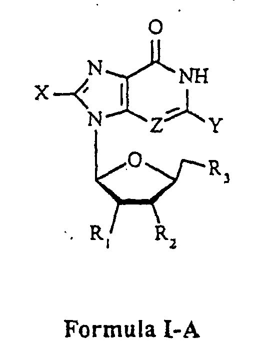 wherein X is selected from, R^{x}, F, Cl, Br, I, N₃, -CN, -OR^{x}, -SR^{x}, -NR^{x}₂, -NHNH₂, -NHOH,-CHO, -CONH₂, -COOR^{x}, and -L-A; where R^{x} is selected from alkyl, alkenyl, alkynyl, and aralkyl, acetyl, acyl, sulfonyl; L is a linker and selected from alkyl, alkenyl, alkynyl, and aralkyl; and A is selected from H, -OR', -SR', -NR'₂, -NHNR'₂, -CHO, -COOR', -CONR'₂, where R' is selected from H, Me, Et, allyl, acetyl, -COCF₃;
Y is selected from H, R^{y}, F, Cl, Br, I, N₃, CN, OR^{y}, SR^{y}, NR^{y}₂, where R^{y} is selected from H, alkyl, alkenyl, alkynyl, and aralkyl, acetyl, acyl, sulfonyl;
Z is N or CH; and
R₁, R₂, and R₃ are independently selected from H, -OH, -OAc, -OBz, -OP(O₂)OH;

2. A compound, a 7-substituted-8-oxo-α-or β- L-guanosine analog, having a structure: according to Figure I-B: 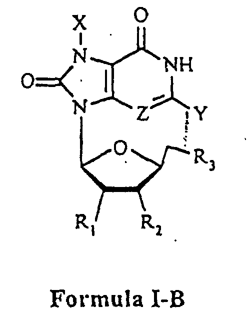 wherein X is selected from H, R^{x}, -NH₂, -CHO, -COOR^{x}, -L-A, where R^{x} is selected from alkyl, alkenyl, alkynyl, and aralkyl; L is a linker and selected from alkyl, alkenyl, alkynyl, and aralkyl; A is selected from H, F, Cl, Br, I, -OR', -SR', -NR'₂, -NHNH₂, -NHOH, N₃, -CHO, -CONH₂, -COOR', -CN, where R' is selected from Me, Et, allyl, acetyl, -COCF₃;
Y is selected from H, R^{y}, F, Cl, Br, I, N₃, -CN, -OR^{y}, -SR^{y}, -NR^{y}₂, where R^{y} is selected from H, alkyl, alkenyl, alkynyl, and aralkyl, acetyl, acyl, sulfonyl;
Z is N or CH;
R₁, R₂, and R₃ are independently selected from H, -OH, -OAc, -OBz, -OP(O₂)OH;
with the proviso that where R₁, R₂, and R₃ are OH, then Z is not N, Y is not NH₂, and X is not propyl.

3. A compound, a 7-deaza-7,8-mono- or disubstituted α-or β-L-guanosine analog, having a structure according to Figure I-C: 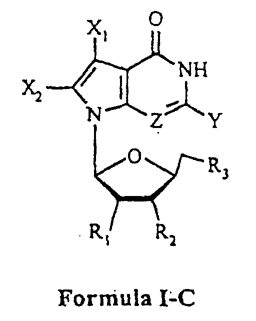 wherein X₁ and X₂ are independently selected from H, R^{x}, F, Cl, Br, I, N₃, -CN, -OR^{x}, -SR^{x}, -NR^{x}₂, -NHNH₂, -NHOH, -CHO, -CONH₂, -COOR^{x}, and -L-A; where R^{x} is selected from alkyl, alkenyl, alkynyl, and aralkyl, acetyl, acyl, sulfonyl; L is a linker and selected from alkyl, alkenyl, alkynyl, and aralkyl; and A is selected from H, -OR', -SR', -NR'₂, -NHNR'₂, -CHO, -COOR', -CONR'₂, where R' is selected from H, Me, Et, allyl, acetyl, -COCF₃;
Y is selected from H, R^{y}, F, Cl, Br, I, N₃, -CN, -OR^{y}, -SR^{y}, -NR^{y}₂, where R^{y} is selected from H, alkyl, alkenyl, alkynyl, and aralkyl, acetyl, acyl, sulfonyl;
Z is N or CH;
R₁, R₂, and R₃ are independently selected from H, -OH, -OAc, -OBz, -OP(O₂)OH.

4. A compound, 7-deaza-8-aza-7- α-or β- L-guanosine analog, having a structure according to Figure I-D: 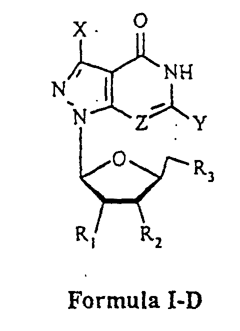 wherein X is selected from H, R^{x}, F, Cl, Br, I, N₃, -CN, -OR^{x}, -SR^{x}, -NR^{x}₂, -NHNH₂, -NHOH,-CHO, -CONH₂, -COOR^{x}, and -L-A; where R^{x} is selected from alkyl, alkenyl, alkynyl, and aralkyl, acetyl, acyl, sulfonyl; L is a linker and selected from alkyl, alkenyl, alkynyl, and aralkyl; and A is selected from H, -OR', SR', -NR'₂, -NHNR'₂, -CHO,-COOR', -CONR'₂, where R' is selected from H, Me, Et, allyl, acetyl, -COCF₃;
Y is selected from H, R^{y}, F, Cl, Br, I, N₃, -CN, -OR^{y}, -SR^{y}, -NR^{y}₂, where R^{y} is selected from H, alkyl, alkenyl, alkynyl, and aralkyl, acetyl, acyl, sulfonyl;
Z is N or CH;
R₁, R₂, and R₃ are independently selected from H, -OH, -OAc, -OBz, -OP(O₂)OH.

5. A compound, a thiazolo[4,5-d]pyrimidine α-or β-L-nucleoside, having a structure according to Figure I-E: 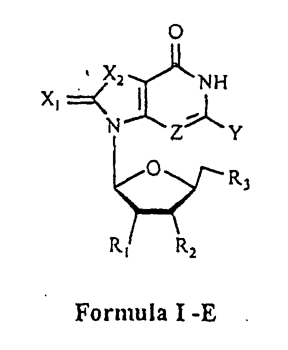 wherein X₁ = O, S, =NH, =NNH₂, =NHOH, =NR^{x} where R^{x} is selected from alkyl, alkyenyl, alkynyl, and aralkyl, acyl;
X₂ is S, O, or Se
Y is selected from H, R^{y}, F, Cl, Br, I, N₃, -CN, -OR^{y}, -SR^{y}, -NR^{y}₂, where R^{y} is selected from H, alkyl, alkenyl, alkynyl, and aralkyl, acetyl, acyl, sulfonyl;
Z is N or CH;
R₁, R₂, and R₃ are independently selected from H, -OH, -OAc, -OBz, -OP(O₂)OH,
with the proviso that when R₁, R₂, and R₃ are OH, then Z is not N, X₁ is not O, X₂ is not S, and Y is not NH₂.

6. A pharmaceutical comprising a compound according to any one of claims 1-5, or a pharmaceutically acceptable ester or salt thereof, admixed with at least one pharmaceutically acceptable carrier.

7. Use of a compound according to any one of claims 1-5 for the manufacture of a pharmaceutical composition for treating a patient having a medical condition selected from the group consisting of an infection, an infestation, a neoplasm or an autoimmune disease.

8. The use of claim 7 wherein the a pharmaceutical composition comprises the compound in a therapeutically effective amount.

9. Use of a compound according to any one of claims 1-5 for the manufacture of a pharmaceutical composition for treatment of a condition associated with an in balance in Th1 and Th2 cytokines.

## Patentansprüche

1. Eine Verbindung, ein 8-substituiertes α- oder β-L-Guanosinanalogon, mit einer Struktur gemäß Formel I-A, 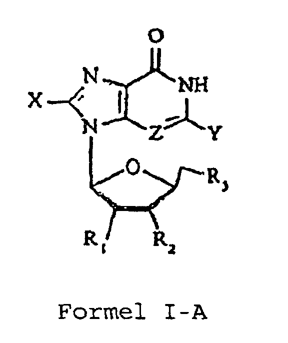 wobei X aus R^{x}, F, Cl, Br, I, N₃, -CN, -OR^{x}, -SR^{x}, -NR^{x}₂, -NHNH₂, -NHOH, -CHO, -CONH₂, -COOR^{x} und -L-A ausgewählt wird; wobei R^{x} aus Alkyl, Alkenyl, Alkynyl und Aralkyl, Acetyl, Acyl, Sulfonyl ausgewählt wird; L ein Linker ist und aus Alkyl, Alkenyl, Alkynyl und Aralkyl ausgewählt wird; und A aus H, -OR', -SR', -NR'₂, -NHNR'₂, -CHO, -COOR', -CONR'₂ ausgewählt wird, wobei R' aus H, Me, Et, Allyl, Acetyl, -COCF₃ ausgewählt wird;
Y aus H, R^{y}, F, Cl, Br, I, N₃, CN, OR^{y}, SR^{y}, NR^{y}₂ ausgewählt wird, wobei R^{y} aus H, Alkyl, Alkenyl, Alkynyl und Aralkyl, Acetyl, Acyl, Sulfonyl ausgewählt wird;
Z -N oder -CH ist; und
R₁, R₂ und R₃ unabhängig aus H, -OH, -OAc, -OBz, -OP(O₂)OH ausgewählt werden.

2. Eine Verbindung, ein 7-substituiertes-8-Oxo-α- oder β-L-Guanosinanalogon, mit einer Struktur gemäß Figur I-B: 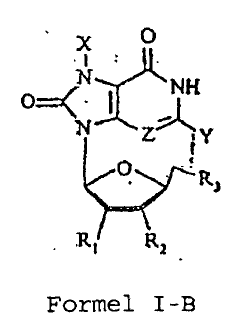 wobei X aus H, R^{x}, -NH₂, -CHO, -COOR^{x}, -L-A ausgewählt wird, wobei R^{x} aus Alkyl, Alkenyl, Alkynyl und Aralkyl ausgewählt wird; L ein Linker ist und aus Alkyl, Alkenyl, Alkynyl und Aralkyl ausgewählt wird; A aus H, F, Cl, Br, I, -OR', -SR', -NR'₂, -NHNH₂, -NHOH, N₃, -CHO, -CONH₂, -COOR', -CN ausgewählt wird, wobei R' aus Me, Et, Allyl, Acetyl, -COCF₃ ausgewählt wird;
Y aus H, R^{y}, F, Cl, Br, I, N₃, -CN, -OR^{y}, -SR^{y}, -NR^{y}₂ ausgewählt wird, wobei R^{y} aus H, Alkyl, Alkenyl, Alkynyl und Aralkyl, Acetyl, Acyl, Sulfonyl ausgewählt wird;
Z -N oder -CH ist;
R₁, R₂ und R₃ unabhängig aus H, -OH, -OAc, -OBz, -OP(O₂)OH ausgewählt werden;
mit der Maßgabe, dass wenn R₁, R₂ und R₃ -OH sind, dann Z nicht N ist, Y nicht NH₂ ist und X nicht Propyl ist.

3. Eine Verbindung, ein 7-Deaza-7,8-mono- oder disubstituiertes α- oder β-L-Guanosinanalogon, mit einer Struktur gemäß Figur I-C: 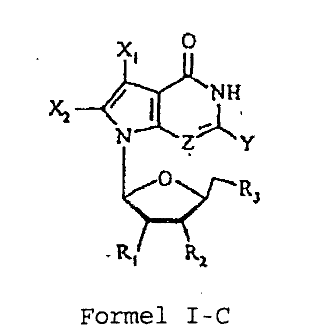 wobei X₁ und X₂ unabhängig aus H, R^{x}, F, Cl, Br, I, N₃, -CN, -OR^{x}, -SR^{x}, -NR^{x}₂, -NHNH₂, -NHOH, -CHO, -CONH₂, -COOR^{x} und -L-A ausgewählt werden; wobei R^{x} aus Alkyl, Alkenyl, Alkynyl und Aralkyl, Acetyl, Acyl, Sulfonyl ausgewählt wird; L ein Linker ist und aus Alkyl, Alkenyl, Alkynyl und Aralkyl ausgewählt wird; und A aus H, -OR', -SR', -NR'₂, -NHNR'₂, -CHO, -COOR', -CONR'₂ ausgewählt wird, wobei R' aus H, Me, Et, Allyl, Acetyl, -COCF₃ ausgewählt wird;
Y aus H, R^{y}, F, Cl, Br, I, N₃, -CN, -OR^{y}, -SR^{y}, -NR^{y}₂ ausgewählt wird, wobei R^{y} aus H, Alkyl, Alkenyl, Alkynyl und Aralkyl, Acetyl, Acyl, Sulfonyl ausgewählt wird;
Z -N oder -CH ist;
R₁, R₂ und R₃ unabhängig aus H, -OH, -OAc, -OBz, -OP(O₂)OH ausgewählt werden.

4. Eine Verbindung, ein 7-Deaza-8-aza-7-α- oder β-L-Guanosinanalogon, mit einer Struktur gemäß Figur I-D: 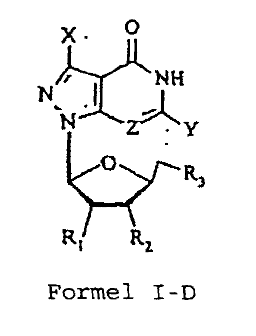 wobei X aus H, R^{x}, F, Cl, Br, I, N₃, -CN, -OR^{x}, -SR^{x}, -NR^{x}₂, -NHNH₂, -NHOH, -CHO, -CONH₂, -COOR^{x} und -L-A ausgewählt wird; wobei R^{x} aus Alkyl, Alkenyl, Alkynyl und Aralkyl, Acetyl, Acyl, Sulfonyl ausgewählt wird; L ein Linker ist und aus Alkyl, Alkenyl, Alkynyl und Aralkyl ausgewählt wird; und A aus H, -OR', -SR', -NR'₂, -NHNR'₂, -CHO, -COOR', -CONR'₂ ausgewählt wird, wobei R' aus H, Me, Et, Allyl, Acetyl, -COCF₃ ausgewählt wird;
Y aus H, R^{y}, F, Cl, Br, I, N₃, -CN, -OR^{y}, -SR^{y}, -NR^{y}₂ ausgewählt wird, wobei R^{y} aus H, Alkyl, Alkenyl, Alkynyl und Aralkyl, Acetyl, Acyl, Sulfonyl ausgewählt wird;
Z -N oder -CH ist;
R₁, R₂ und R₃ unabhängig aus H, -OH, -OAc, -OBz, -OP(O₂)OH ausgewählt werden.

5. Eine Verbindung, ein Thiazolo[4,5-d]pyrimidin α- oder β-L-Nucleosid, mit einer Struktur gemäß Figur I-E: 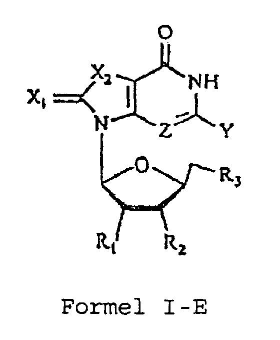 wobei X₁ = O,S, = NH, = NNH₂, = NHOH, = NR^{x}, wobei R^{x} aus Alkyl, Alkenyl, Alkynyl und Aralkyl, Acyl ausgewählt wird;
X₂ -S, -O oder -Se ist;
Y aus H, R^{y}, F, Cl, Br, I, N₃, -CN, -OR^{y}, -SR^{y}, -NR^{y}₂ ausgewählt wird, wobei R^{y} aus H, Alkyl, Alkenyl, Alkynyl und Aralkyl, Acetyl, Acyl, Sulfonyl ausgewählt wird;
Z -N oder -CH ist;
R₁, R₂ und R₃ unabhängig aus H, -OH, -OAc, -OBz, -OP(O₂)OH ausgewählt werden;
mit der Maßgabe, dass wenn R₁, R₂ und R₃ -OH sind, dann Z nicht N ist, X₁ nicht O ist, X₂ nicht S ist und Y nicht NH₂ ist.

6. Ein Arzneimittel, das eine Verbindung gemäß einem der Ansprüche 1-5 oder einen pharmazeutisch geeigneten Ester oder ein Salz davon, vermischt mit wenigstens einem pharmazeutisch geeigneten Träger, umfasst.

7. Verwendung einer Verbindung gemäß einem der Ansprüche 1-5 für die Herstellung einer pharmazeutischen Zusammensetzung zur Behandlung eines Patienten mit einem medizinischen Zustand, der aus der Gruppe, die aus einer Infektion, einem Befall, einem Geschwulst oder einer Autoimmunerkrankung besteht, ausgewählt wird.

8. Die Verwendung nach Anspruch 7, wobei die pharmazeutische Zusammensetzung die Verbindung in einer therapeutisch wirksamen Menge umfasst.

9. Verwendung einer Verbindung gemäß einem der Ansprüche 1-5 für die Herstellung einer pharmazeutischen Zusammensetzung für die Behandlung eines Zustandes, der mit einem Ungleichgewicht in den Th1 und Th2 Cytokinen verbunden ist.

## Revendications

1. Composé d'un analogue d'une α - ou d'une β -L-guanosine substituée en 8, ayant une structure selon la formule I-A : 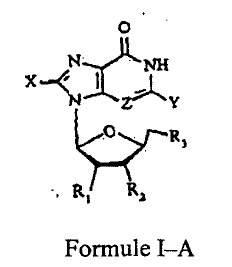 dans laquelle X est choisi parmi les éléments suivants : R^{X}, F, Cl, Br, I, N₃, -CN,-OR^{X}, -SR^{X}, -NR^{X}₂, -NHNH₂, -NHOH, -CHO, -CONH₂, -COOR^{X}, et -L-A ; dans lesquels R^{x} est choisi parmi un groupe alkyle, alcényle, alcynyle, et un groupe aralkyle, acétyle, acyle, sulfonyle ; L est un lieur et est choisi parmi un groupe alkyle, alcényle, alcynyle, et aralkyle ; et A est choisi parmi les éléments suivants : H, -OR', -SR', -NR'₂, -NHNR'₂ ; -CHO, -COOR', -CONR'₂ ; dans lesquels R' est choisi parmi les éléments suivants : H, Me, Et, un groupe allyle, un groupe acétyle, -COCF₃ ;
Y est choisi parmi les éléments suivants : H, R^{Y}, F, Cl, Br, I, N₃, CN, OR^{Y}, SR^{Y}, NR^{Y}₂ ; dans lesquels R^{Y} est choisi parmi les éléments suivants : H, un groupe alkyle, alcényle, alcynyle, et un groupe aralkyle, acétyle, acyle, sulfonyle ;
Z est N ou CH ; et
R₁, R₂ et R₃ sont choisis indépendamment parmi les éléments suivants : H, -OH, -OAc, -OBz, -OP(O₂)OH.

2. Composé d'un analogue d'une 8-oxo- α - ou d'une 8-oxo-β-L-guanosine substituée en 7, ayant une structure selon la formule I-B : 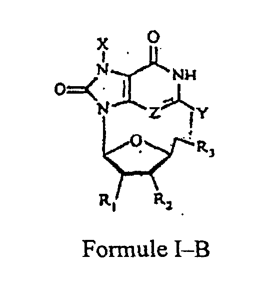 dans laquelle X est choisi parmi les éléments suivants : H, R^{X}, -NH₂, -CHO,-COOR^{X}, et -L-A ; dans lesquels R^{X} est choisi parmi un groupe alkyle, alcényle, alcynyle, et un groupe aralkyle ; L est un lieur et est choisi parmi un groupe alkyle, alcényle, alcynyle, et aralkyle ; A est choisi parmi les éléments suivants : H, F, CI, Br, I, -OR', -SR', -NR'₂, -NHNH₂, -NHOH, N₃, -CHO, -CONH₂, -COOR', -CN ; dans lesquels R' est choisi parmi les éléments suivants : Me, Et, un groupe allyle, un groupe acétyle, -COCF₃ ;
Y est choisi parmi les éléments suivants : H, R^{Y}, F, Cl, Br, I, N₃, -CN, -OR^{Y}, -SR^{Y}, -NR^{Y}₂ ; dans lesquels R^{Y} est choisi parmi les éléments suivants : H, un groupe alkyle, alcényle, alcynyle, et un groupe aralkyle, acétyle, acyle, sulfonyle ;
Z est N ou CH ;
R₁, R₂ et R₃ sont choisis indépendamment parmi les éléments suivants : H, -OH, -OAc, -OBz, -OP(O₂)OH ;
à la condition que là où R₁, R₂ et R₃ sont OH, alors Z n'est pas N, Y n'est pas NH₂, et X n'est pas un groupe propyle.

3. Composé d'un analogue d'une 7-déaza-7,8-mono- ou disubstituée α- ou β-L-guanosine, ayant une structure selon la formule I-C : 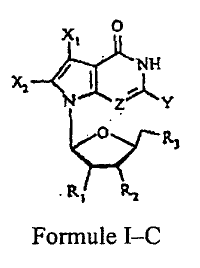 dans laquelle X₁ et X₂ sont choisis indépendamment parmi les éléments suivants : H, R^{X}, F, Cl, Br, I, N₃, -CN, -OR^{X}, -SR^{X}, -NR^{X}₂, -NHNH₂, -NHOH, -CHO, -CONH₂, -COOR^{X}, et L-A ; dans lesquels R^{X} est choisi parmi un groupe alkyle, alcényle, alcynyle, et un groupe aralkyle, acétyle, acyle, sulfonyle ; L est un lieur et est choisi parmi un groupe alkyle, alcényle, alcynyle, et aralkyle ; et A est choisi parmi les éléments suivants : H, -OR', -SR', -NR'₂, -NHNR'₂, -CHO, -COOR', -CONR'₂ ; dans lesquels R' est choisi parmi les éléments suivants : H, Me, Et, un groupe allyle, un groupe acétyle, -COCF₃ ;
Y est choisi parmi les éléments suivants : H, R^{Y}, F, Cl, Br, I, N₃, -CN, -OR^{Y}, -SR^{Y}, -NR^{Y}₂ ; dans lesquels R^{Y} est choisi parmi les éléments suivants : H, un groupe alkyle, alcényle, alcynyle, et un groupe aralkyle, acétyle, acyle, sulfonyle ;
Z est N ou CH ;
R₁, R₂, et R₃ sont choisis indépendamment parmi les éléments suivants : H, -OH, -OAc, -OBz, -OP(O₂)OH.

4. Composé d'un analogue d'une 7-déaza-8-aza-7-α- ou β-L-guanosine, ayant une structure selon la formule I-D : 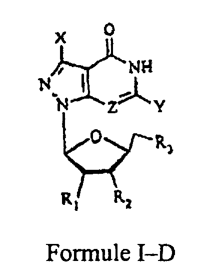 dans laquelle X est choisi parmi les éléments suivants : H, R^{X}, F, Cl, Br, I, N₃, -CN, -OR^{X}, -SR^{X}, -NR^{X}₂, -NHNH₂, -NHOH, -CHO, -CONH₂, -COOR^{X}, et L-A ; dans lesquels R^{X} est choisi parmi un groupe alkyle, alcényle, alcynyle, et un groupe aralkyle, acétyle, acyle, sulfonyle ; L est un lieur et est choisi parmi un groupe alkyle, alcényle, alcynyle, et aralkyle ; et A est choisi parmi les éléments suivants : H, -OR', -SR', -NR'₂, -NHNR'₂, -CHO, -COOR', -CONR'₂ ; dans lesquels R' est choisi parmi les éléments suivants : H, Me, Et, un groupe allyle, un groupe acétyle, -COCF₃;
Y est choisi parmi les éléments suivants : H, R^{Y}, F, Cl, Br, I, N₃, -CN, -OR^{Y}, -SR^{Y}, -NR^{Y}₂ ; dans lesquels R^{Y} est choisi parmi les éléments suivants : H, un groupe alkyle, alcényle, alcynyle, et un groupe aralkyle, acétyle, acyle, sulfonyle ;
Z est N ou CH ;
R₁, R₂, et R₃ sont choisis indépendamment parmi les éléments suivants : H, -OH, -OAc, -OBz, -OP(O₂)OH.

5. Composé d'un thiazolo-[4,5-d]-pyrimidine α- ou β-L-nucléoside, ayant une structure selon la formule I-E : 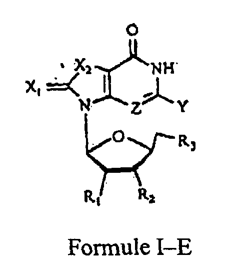 dans laquelle X₁ = O, S, =NH, =NNH₂, =NHOH, =NR^{x} dans lesquels R^{X} est choisi parmi un groupe alkyle, alcényle, alcynyle, et un groupe aralkyle, acyle ;
X₂ est S, O ou Se ;
Y est choisi parmi les éléments suivants : H, R^{Y}, F, Cl, Br, I, N₃, -CN, -OR^{Y}, -SR^{Y}, NR^{Y}₂ ; dans lesquels R^{Y} est choisi parmi les éléments suivants : H, un groupe alkyle, alcényle, alcynyle, et un groupe aralkyle, acétyle, acyle, sulfonyle ;
Z est N ou CH ;
R₁, R₂, et R₃ sont choisis indépendamment parmi les éléments suivants : H, -OH, -OAc, -OBz, -OP(O₂)OH ;
à la condition que quand R₁, R₂, et R₃ sont -OH, alors Z n'est pas N, X₁ n'est pas O, X₂ n'est pas S, et Y n'est pas NH₂.

6. Médicament comprenant un composé selon l'une quelconque des revendications 1 à 5, ou un de ses esters ou de ses sels pharmaceutiquement acceptable, ajouté à au moins un véhicule pharmaceutiquement acceptable.

7. Utilisation d'un composé selon l'une quelconque des revendications 1 à 5, pour la fabrication d'une composition pharmaceutique pour traiter un patient ayant une affection médicale choisie dans le groupe constitué d'une infection, d'une infestation, d'un néoplasme ou d'une maladie auto-immune.

8. Utilisation selon la revendication 7, dans laquelle la composition pharmaceutique comprend le composé en une quantité thérapeutiquement efficace.

9. Utilisation d'un composé selon l'une quelconque des revendications 1 à 5, pour la fabrication d'une composition pharmaceutique pour le traitement d'une affection associée avec un déséquilibre de cytokines Th1 et Th2.
